# EUROPEAN PATENT SPECIFICATION

(11) **EP 3 400 029 B1**
(45) Date of publication and mention of the grant of the patent: **02.03.2022**
(21) Application number: 17735891.8
(22) Date of filing: 06.01.2017
(51) Int. Cl.: A61K 38/36, A61L 26/00, A61L 24/10, A61K 35/14, A61K 38/48, A61L 24/00, A61P 17/02, A61P 7/04

(54) **COMPOSITIONS FOR REDUCING TISSUE ADHESIONS**
ZUSAMMENSETZUNGEN ZUR REDUZIERUNG VON GEWEBEADHÄSION
COMPOSITIONS POUR RÉDUIRE LES ADHÉSIONS TISSULAIRES

(30) Priority: 07.01.2016 US 201662275783 P; 08.04.2016 US 201662319807 P
(43) Date of publication of application: 14.11.2018
(73) Proprietor: Eio Biomedical Ltd, 7636015 Nazareth (IL)
(72) Inventor: PILPEL, Yair, 7655923 Rehovot (IL)
(74) Representative: ABG Intellectual Property Law, S.L.
(86) International application number: PCT/IB2017/000060
(87) International publication number: WO 2017/118910

(56) References cited:
- WO-A1-2014/119836
- US-A- 4 981 952
- US-A- 5 445 958
- US-A- 5 939 325
- US-A- 6 099 837
- US-A1- 2003 211 591
- US-A1- 2004 005 350
- US-A1- 2009 069 543
- US-A1- 2009 318 843
- US-A1- 2013 058 906
- US-A1- 2014 154 233
- US-A1- 2015 174 215
- US-B1- 6 613 325
- WARD B C ET AL: "Abdominal Adhesions: Current and Novel Therapies", JOURNAL OF SURGICAL RESEARCH, ACADEMIC PRESS INC., SAN DIEGO, CA, US, vol. 165, no. 1, 1 January 2011 (2011-01-01), pages 91-111, XP027553743, ISSN: 0022-4804 [retrieved on 2009-10-02]
- TOPAL E ET AL: "A comparison of three fibrinolytic agents in prevention of intra-abdominal adhesions", ACTA CHIRURGICA BEL, PUBLICATIONS "ACTA MEDICA BELGICA"., BRUSSELS, BE, vol. 110, no. 1, 1 January 2010 (2010-01-01), pages 71-75, XP009186237, ISSN: 0001-5458, DOI: 10.1080/00015458.2010.11680569
- ANTHONY IMUDIA ET AL: "Pathogenesis of Intra-abdominal and Pelvic Adhesion Development", SEMINARS IN REPRODUCTIVE MEDICINE, vol. 26, no. 04, 1 July 2008 (2008-07-01), pages 289-297, XP055608922, US ISSN: 1526-8004, DOI: 10.1055/s-0028-1082387

## Description

### FIELD OF THE INVENTION

Disclosed herein are compositions and uses thereof for reducing adhesions severity resulting from wounds and surgery.

### BACKGROUND OF THE INVENTION

Abdominal surgery constitutes a significant traumatic event to the peritoneal cavity. The natural response of the body to the trauma is an immediate inflammatory response, which stimulates the activities of several cell types including, among others, fibroblasts, endothelial cells and immune cells. Without intending to be limited to any particular theory, peritoneal adhesions are formed as a consequence of the excessive activity of these cells, which produce significant amounts of extracellular matrix proteins and initiate the formation of connective tissue. Postoperative adhesions, which are believed to manifest themselves in the majority of patients within one week following intraabdominal surgery but persist for years thereafter, effectively becoming a congruent part of the body, can result in serious medical consequences including, *inter alia,* pain, secondary infertility and small bowel obstruction. Although adhesions are common in intraabdominal surgeries, adhesions can develop in all types of surgeries.

### BRIEF DESCRIPTION OF THE FIGURES

The present invention will be further explained with reference to the attached drawings, wherein like structures are referred to by like numerals throughout the several views. The drawings shown are not necessarily to scale, with emphasis instead generally being placed upon illustrating the principles of the present invention. Further, some features may be exaggerated to show details of particular components.

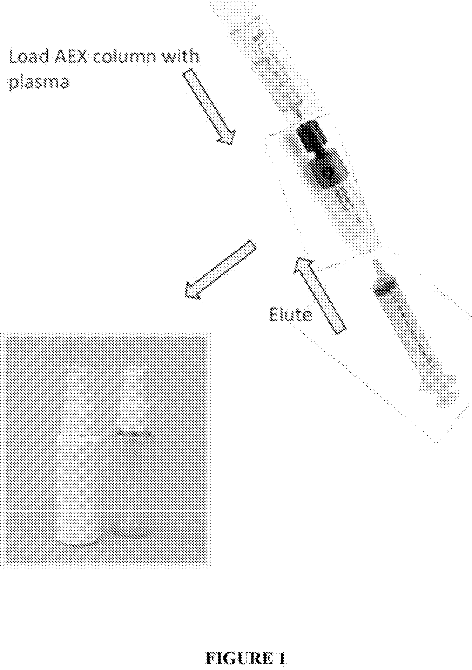

Figure 1 provides an embodiment of the kit and method of the present invention, showing the steps required while using the kit to prepare a composition according to some embodiments of the present invention following plasma collection.
Figures 2A-2C are photographs of the abdominal cavity of rats that have undergone a cecal abrasion and peritoneal sidewall defect surgical manipulation, at two weeks after treatment, after topical administration of an embodiment of the composition of the present invention compared with a saline control.
Figures 3A-F show quantitative results of an experiment directed to analyzing adhesions in rats after treatment with some embodiments of the composition of the present invention.
Figures 4A-C show quantitative results of an experiment measuring adhesion area, grade, or score in rats after treatment with some embodiments of the composition of the present invention.
Figure 5 shows a photograph of an embodiment of the kit of the present invention.
Figures 6, 7, and 8 show illustrations of embodiments of the kit of the present invention.
Figures 9A to 9H show graphed calibration curves of clotting factors: Factor II (Figures 9A and 9B), Factor V (Figure 9C), Factor VII (Figure 9D), Factor X (Figures 9E and 9F), and Fibrinogen (Figures 9G (log curve) and 9H (linear fit)), according to some embodiments of the composition of the present invention.
Figure 10 shows a graphical illustration directed to the effects of increasing salt concentration of some embodiments of the composition of the present invention.

### SUMMARY OF THE INVENTION

In one aspect, the present invention provides a purified composition, comprising:
proaccelerin (factor V), prothrombin (factor II), proconvertin (factor VII), and
Stuart-Prower factor (factor X),
   wherein the concentration of proaccelerin is from 300% to 2000% relative to the concentration in a normal blood plasma sample, wherein the concentration is determined by extrapolating the activity of proaccelerin using a clotting assay and plotting a standard curve;
   wherein the concentration of each one of proconvertin, prothrombin and Stuart-Prower factor is from 500% to 2500% or from 150% to 2000% relative to the concentration in a normal blood plasma sample; and
   wherein the composition is a fibrinogen-depleted plasma-derived concentrate.

In some embodiments, the composition comprises 1-5% fibrinogen relative to total amount of fibrinogen in the blood plasma.

In another aspect, the present invention provides the composition according to the other aspect of the present invention for use in reducing adhesion severity by topical application at a surgical site in a subject undergoing surgery, wherein the composition is an aqueous composition.

### DETAILED DESCRIPTION OF THE INVENTION

Provided herein are compositions useful in reducing or preventing tissue adhesions at surgical sites. The compositions are based on a composition according to some embodiments of the present invention. Without being bound to theory, such a composition rapidly activates on bleeding and raw, injured tissue, due to the presence and exposure of Tissue Factor (TF) (also referred to herein as "clotting factor") protein in damaged tissue. TF is a strong direct activator of the coagulation factor VII (FVII), and is a main activator of the popularly referred to "extrinsic system" of blood coagulation.

The clotting factors include, but are not limited to, proaccelerin (factor V, FV), prothrombin (factor II, FII), proconvertin (factor VII, FVII), Stuart-Prower factor (factor X, FX), and fibrin stabilizing factor (factor XIII, FXIII).

### Definitions

As used herein, the singular forms "a", "an" and "the" include plural forms unless the content clearly dictates otherwise. Where aspects or embodiments are described in terms of Markush groups or other grouping of alternatives, those skilled in the art will recognize that the invention is also thereby described in terms of any individual member or subgroup of members of the group.

As used herein, the terms "comprising", "including", "having" and grammatical variants thereof are to be taken as specifying the stated features, steps or components but do not preclude the addition of one or more additional features, steps, components or groups thereof.

When a numerical value is preceded by the term "about", the term "about" is intended to indicate +/-10%.

As used here, "treating a subject" refers to administering to the subject a substance effective to ameliorate symptoms associated with a disorder or disease, to lessen the severity of the disorder or disease, to cure the disorder or disease, to slow down the progression of the disorder or disease, or to delay the onset of the disorder or disease. "Treatment" refers to both therapeutic treatment and prophylactic or preventative measures, wherein the object is to prevent a disorder, to delay the onset of the disorder or reduce the symptoms of a disorder. Those in need of treatment include those already experiencing the disease or condition, those prone to having the disease or condition, and those in which the disease or condition is to be prevented. The compositions disclosed herein are administered before onset of the disorder or symptoms, e.g., but not limited to, to prevent or reduce tissue adhesion.

The presence of a composition comprising clotting factors (especially those that are activated either directly or indirectly by Factors, e.g., but not limited to, Factors VII, X, II, and V) in such cases where two opposing sides of a wound are physically forced together (as with stitches, staples, tape, etc.) would enhance the clotting at the site and facilitate faster healing. Therefore some embodiments of the composition of the present invention may be used for improving the healing of external wounds (e.g., but not limited to, abdominal surgery, e.g., but not limited to, C-section surgery).

As used herein, a "therapeutically effective amount" refers to an amount of the compound or component which is effective to achieve an improvement in a subject or his physiological systems including, but not limited to, elimination of symptoms, delayed onset of a disorder, slower progress of symptoms and other indicators selected as appropriate by those skilled in the art.

As used herein, the term "um" or "µm" refer to micron or micrometer, "g" refers to gram, "mg" refers to milligram, "ml" refers to milliliter and "L" refers to liter.

As used herein, "plasma", "blood plasma", and "blood plasma sample" are used interchangeably and refer to the plasma fraction of blood that contains, *inter alia,* salts, enzymes, and proteins including immunoglobulins (antibodies), clotting factors and albumin, lipids and microvesicles, and fibrinogen, and which may also contain platelets and platelet fragments. A "plasma source" may be, but is not limited to, plasma from autologous or single-donor blood, plasma from fractionation, pooled plasma, cryo-poor plasma, recovered plasma, and plasma which is the fluid portion of human blood collected by plasmapheresis.

As used herein, a "plasma derived concentrate" or "plasma derived concentrate composition" refers to plasma that has been treated to retain certain blood proteins including clotting factors in a concentrated form. Without wishing to be bound to theory, the composition disclosed herein may improve the adhesion prevention activity by providing a thin layer, local clot on sites of extravasation and bleeding.

As used herein, a "fibrinogen-depleted plasma-derived concentrate" or "fibrinogen-depleted plasma-derived composition" or "Extrinsic System Concentrate [ESC]" refers to plasma that has been treated to remove most of the fibrinogen and retain certain blood proteins including clotting factors in a concentrated form. "Extrinsic System Concentrate" (or "ESC") refers to the same composition as the majority of the Extrinsic System coagulation factors are retained in this composition. Without wishing to be bound to theory, fibrinogen is mostly depleted (e.g., 1 - 5% fibrinogen remaining from total amount of fibrinogen in plasma) from the plasma in order for only endogenous fibrinogen released in sites of injury, trauma, or bleeding, to be used as substrate. Several methods to obtain fibrinogen-depleted plasma may be used. One is by the use of a biocompatible polymer such as PEG, dextran, chondroitin sulfate, heparin, hyaluronic acid etc., in the blood followed by the centrifugation step that is carried out to sediment the cellular fraction of the blood and precipitate fibrinogen. As only the soluble upper plasma layer is collected for further processing, the precipitated fibrinogen is removed. Another method comprises utilizing the step of the anion exchange-based purification and concentration of clotting factors from the plasma. An optional wash step may be altered to control the level of depletion of the less tightly bound fibrinogen while retaining other, more tightly bound clotting factors for subsequent elution. It is recognized that under some conditions, it may be advantageous to retain a certain amount of Fibrinogen. Without wishing to be bound to theory, this small amount of Fibrinogen may serve as a nucleation core for endogenous Fibrinogen found on site in places prone to adhesion formation, but would not in itself be sufficient to form a Fibrin network that would initiate adhesion formation in sites that are not compromised.

As used herein, "purified" refers to a composition subjected to at least one physical or chemical modification. A non-limiting example of a modification is filtration.

As used herein, "essentially free of red and white blood cells" refers to less than 0.5% of the red and/or white blood cells, e.g., less than 0.1% of the red and/or white blood cells as compared to the starting material.

As used herein, "essentially particle free" refer to a level of less than 0.5%, e.g., less than 0.1% of particles of a diameter greater than 0.22 microns or 0.1 microns. As a non-limiting example, particles refer to microvesicles of any source in the blood (e.g. cardiac cell derived, platelet derived, phospholipid micelles, and the like).

Whereas the smallest cells found in the blood (platelets) are on average about 3-5 microns in diameter, some microvesicles are smaller than or equal to 200 nm (0.2 microns). The main exclusion of microvesicles is carried out by filtration, and therefore the pore size of the filter would determine the size and relative amounts of microvesicles that would remain in the preparation. Microvesicles have been reported in the ranges of 100-500 nm (Zubairova LD et al., Sci Rep. 2015 5:17611.), and TF containing microvesicles have been characterized using ultracentrifugation to be roughly 200-400 nm in size (Ettelaie C et al., J Extracell Vesicles. 2014. Vol. 3: 23592.).

As used herein, the expression "autologous blood" refers to a composition or system or method wherein a single donor's blood, tissue and/or cell is used and wherein the blood, blood plasma, tissue and/or cells extracted from this donor is intended for use on the same donor. "Allogeneic" methods are using blood, blood plasma, tissue and/or cells from one or more third parties for use on a donor. An autologous product avoids some of the risks associated with the use of biological materials from third parties. In cases where autologous blood cannot be collected it is preferable to use blood from a single donor. The single donor may be screened for potential contamination with hepatitis, HIV, prion, Creutzfeld-Jacob's disease and the like. Allogeneic pooled plasma can be used if pre-treated to remove dangerous viruses and other pathogens such as by solvent-detergent treatment (e.g., but not limited to, Octaplas^{®} commercially available from Octapharma). In cases where single donor blood or plasma is used, similar viral inactivation steps using solvent-detergent treatment may also be performed *on site,* using non-ionic solvents and detergents. Due to their non-ionic nature, these detergents will not bind to an anion exchange column and can be removed by washing (see, as a non-limiting example Gebauer et al., J Chromatogr A. 1999;852(1):83-6). As non-limiting examples, the donor blood or plasma can be obtained from donations (paid and/or unpaid) or collections by a third party, etc.

As used herein, a "tissue adhesion" refers to abnormal fibrous bands that form between tissues and organs, and typically occurs when two injured tissue/organ surfaces are in proximity. The injury often causes inflammation and the deposition of fibrin onto the damaged tissue. The fibrin acts as a hemostat to seal the damaged tissue and cells such as macrophages, fibroblasts and endothelial cells, among others, penetrate into the fibrinous clot, and secrete collagen and other matrix substances to form a permanent fibrous adhesion. Although not all adhesions are detrimental, some can displace internal organs (e.g. ovaries or fallopian tubes, causing secondary infertility), or form a ligature around small bowel loops (causing pathological small bowel obstruction), or cause other complications.

As used herein, a "barrier" or "adhesion barrier" as used herein refers to a pharmaceutical composition or medical implant used to separate the internal tissues and organs while they heal.

### A composition comprising factor V

In one aspect, the present invention provides a composition, comprising:
proaccelerin (factor V), prothrombin (factor II), proconvertin (factor VII), and
Stuart-Prower factor (factor X),
   wherein the concentration of proaccelerin is from 300% to 2000% relative to the concentration in a normal blood plasma sample, wherein the concentration is determined by extrapolating the activity of proaccelerin using a clotting assay and plotting a standard curve;
   wherein the concentration of each one of proconvertin, prothrombin and Stuart-Prower factor is from 500% to 2500% or from 150% to 2000% relative to the concentration in a normal blood plasma sample; and
   wherein the composition is a fibrinogen-depleted plasma-derived concentrate.

In some embodiments, the composition is a plasma derived concentrate.

In some embodiments, an amount of fibrinogen in the composition is reduced by 5% to 95% of an amount of fibrinogen in the blood plasma. In some embodiments, an amount of fibrinogen in the composition is reduced by 25% to 75% of an amount of fibrinogen in the blood plasma. In some embodiments, the amount of fibrinogen in the composition is tested using a Clauss assay.In some embodiments, the composition is a fibrinogen-depleted plasma-derived concentrate.

In some embodiments, the composition according to some embodiments of the present invention, further comprises: fibrin stabilizing factor (factor XIII), wherein an amount of the fibrin stabilizing factor in the composition is between 0.01 IU/mL to 100 IU/mL.

In some embodiments, the amount of the prothrombin in the composition is between 500% to 2500% compared to the amount of prothrombin in a normal blood plasma sample.

In some embodiments, the amount of the proconvertin in the composition is between 500% to 2500% compared to the amount of proconvertin in a normal blood plasma sample.

In some embodiments, the amount of the Stuart-Prower factor in the composition is between 500% to 2500% compared to the amount of Stuart-Prower factor in a normal blood plasma sample.

In some embodiments, the amount of the proaccelerin in the composition is between 300% to 750% compared to the amount of proaccelerin in a normal blood plasma sample. In some embodiments, the amount of the proaccelerin in the composition is between 375% to 750% compared to the amount of proaccelerin in a normal blood plasma sample. In some embodiments, the amount of the proaccelerin in the composition is between 450% to 750% compared to the amount of proaccelerin in a normal blood plasma sample. In some embodiments, the amount of the proaccelerin in the composition is between 525% to 750% compared to the amount of proaccelerin in a normal blood plasma sample. In some embodiments, the amount of the proaccelerin in the composition is between 600% to 750% compared to the amount of proaccelerin in a normal blood plasma sample. In some embodiments, the amount of the proaccelerin in the composition is between 675% to 750% compared to the amount of proaccelerin in a normal blood plasma sample.

In some embodiments, the amount of the proaccelerin in the composition is between 300% to 525% compared to the amount of proaccelerin in a normal blood plasma sample. In some embodiments, the amount of the proaccelerin in the composition is between 375% to 450% compared to the amount of proaccelerin in a normal blood plasma sample.

In some embodiments, the amount of each one of prothrombin (factor II), proconvertin (factor VII), and Stuart-Prower factor (factor X) is from 150% to 2000% compared to the amount in a normal blood plasma sample. In some embodiments, the amount of each one of said factors is from 150% to 1500% compared to the amount in a normal blood plasma sample. In some embodiments, the amount of each one of said factors is from 150% to 1000% compared to the amount in a normal blood plasma sample. In some embodiments, the amount of the each one of said factors is from 150% to 500% compared to the amount in a normal blood plasma sample. In some embodiments, the amount of the each one of said factors is from 500% to 2500% compared to the amount in a normal blood plasma sample. In some embodiments, the amount of the each one of said factors is from 1000% to 2000% compared to the amount in a blood plasma sample.

In some embodiments, an amount of fibrinogen in the composition is reduced by 5% to 95% of an amount of fibrinogen in the blood plasma. In some embodiments, an amount of fibrinogen in the composition is reduced by 10% to 95% of an amount of fibrinogen in the blood plasma. In some embodiments, an amount of fibrinogen in the composition is reduced by 20% to 95% of an amount of fibrinogen in the blood plasma. In some embodiments, an amount of fibrinogen in the composition is reduced by 30% to 95% of an amount of fibrinogen in the blood plasma. In some embodiments, an amount of fibrinogen in the composition is reduced by 40% to 95% of an amount of fibrinogen in the blood plasma. In some embodiments, an amount of fibrinogen in the composition is reduced by 50% to 95% of an amount of fibrinogen in the blood plasma. In some embodiments, an amount of fibrinogen in the composition is reduced by 60% to 95% of an amount of fibrinogen in the blood plasma. In some embodiments, an amount of fibrinogen in the composition is reduced by 70% to 95% of an amount of fibrinogen in the blood plasma. In some embodiments, an amount of fibrinogen in the composition is reduced by 80% to 95% of an amount of fibrinogen in the blood plasma. In some embodiments, an amount of fibrinogen in the composition is reduced by 90% to 95% of an amount of fibrinogen in the blood plasma.

In some embodiments, an amount of fibrinogen in the composition is reduced by 5% to 905% of an amount of fibrinogen in the blood plasma. In some embodiments, an amount of fibrinogen in the composition is reduced by 5% to 80% of an amount of fibrinogen in the blood plasma. In some embodiments, an amount of fibrinogen in the composition is reduced by 5% to 70% of an amount of fibrinogen in the blood plasma. In some embodiments, an amount of fibrinogen in the composition is reduced by 5% to 60% of an amount of fibrinogen in the blood plasma. In some embodiments, an amount of fibrinogen in the composition is reduced by 5% to 50% of an amount of fibrinogen in the blood plasma. In some embodiments, an amount of fibrinogen in the composition is reduced by 5% to 40% of an amount of fibrinogen in the blood plasma. In some embodiments, an amount of fibrinogen in the composition is reduced by 5% to 30% of an amount of fibrinogen in the blood plasma. In some embodiments, an amount of fibrinogen in the composition is reduced by 5% to 20% of an amount of fibrinogen in the blood plasma. In some embodiments, an amount of fibrinogen in the composition is reduced by 5% to 10% of an amount of fibrinogen in the blood plasma.

In some embodiments, an amount of fibrinogen in the composition is reduced by 10% to 90% of an amount of fibrinogen in the blood plasma. In some embodiments, an amount of fibrinogen in the composition is reduced by 15% to 85% of an amount of fibrinogen in the blood plasma. In some embodiments, an amount of fibrinogen in the composition is reduced by 25% to 75% of an amount of fibrinogen in the blood plasma. In some embodiments, an amount of fibrinogen in the composition is reduced by 35% to 65% of an amount of fibrinogen in the blood plasma. In some embodiments, an amount of fibrinogen in the composition is reduced by 45% to 55% of an amount of fibrinogen in the blood plasma.

In some embodiments, the amount of fibrinogen in the composition is tested using a Clauss assay.

In some embodiments, the concentration of fibrin stabilizing factor in the composition is between 0.01 IU/mL (international units/milliliter) to 100 IU/mL. In some embodiments, the concentration of fibrin stabilizing factor in the composition is between 0.05 IU/mL to 25 IU/mL. In some embodiments, the concentration of fibrin stabilizing factor in the composition is between 0.1 IU/mL to 10 IU/mL. In some embodiments, the concentration of fibrin stabilizing factor in the composition is between 0.5 IU/mL to 5 IU/mL. In some embodiments, the concentration of fibrin stabilizing factor in blood is about 1 IU/mL.

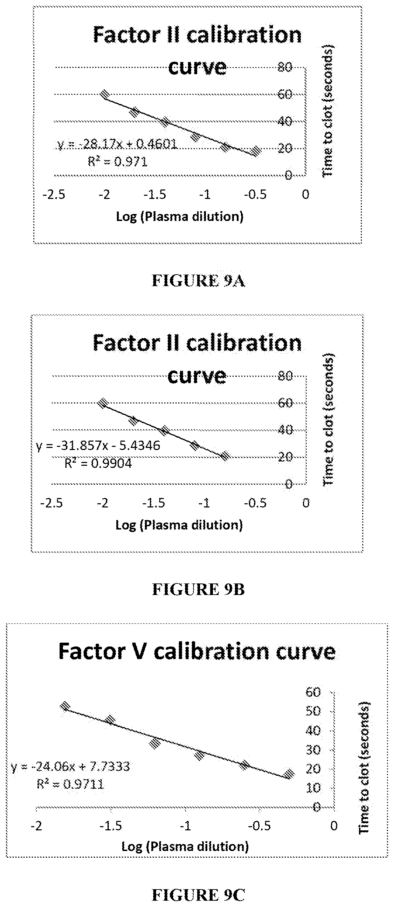

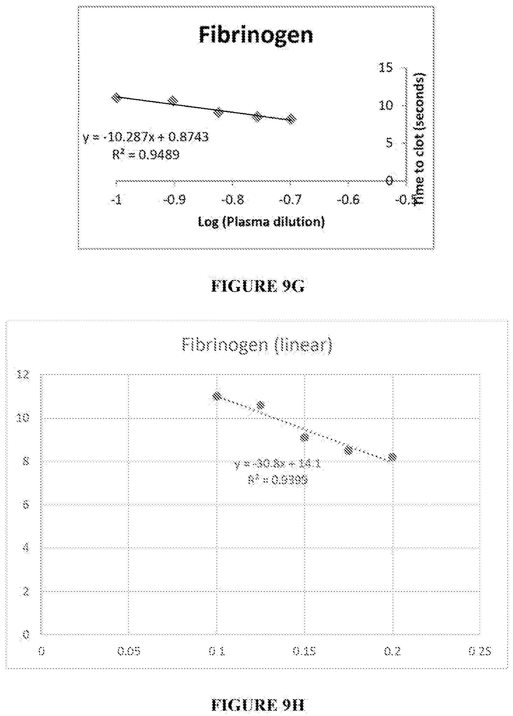

In some embodiments, the concentration of any of the factors (e.g., but not limited to, fibrinogen, proaccelerin, prothrombin, proconvertin, Steward-Prower factor, or fibrin stabilizing factor, or any combination thereof) is determined by extrapolating the activity of the tissue factor using a clotting assay and plotting a standard curve. Examples of standard curves of fibrinogen, proaccelerin, prothrombin, proconvertin, and Steward-Prower factor are shown in Figures 9A-9H.

In some embodiments, an increase in the concentration of any one of the factors results in an increase in an activity of that factor. In a non-limiting example, a concentration of prothrombin in the composition is 250% of a concentration of prothrombin in the blood plasma, and thus the prothrombin activity of the composition is 2.5 times the prothrombin activity in the blood plasma.

In some embodiments, the overall concentration of the clotting factors using this method can reach as much as 20:1 or higher, over what is found in normal blood. In some embodiments, a concentration factor of about 1.5 to 20:1 may be achieved. In some embodiments, the concentration factor is maintained at about 3:1 to 10:1, or about 4:1 to 8:1, or about 5:1 to 6:1. The main considerations that would modify this ratio are: (i) the type of anion exchange resin utilized and the salt concentration required to efficiently elute the coagulation factors therefrom, (ii) when using autologous blood, the amount of initial blood that may be taken from the patient and would be dependent on patient health, and the nature of the surgical procedure to be performed, and (iii) the final volume of extract required to cover the surgical field in view of the amount of blood plasma available for processing, e.g., the amount found in a single plasma donation.

Without being bound to theory, factor V is a cofactor that accelerates the conversion of factor II to factor IIa (i.e., Prothrombin to Thrombin).

. Without being bound to theory, factor VII is the critical factor required for the activation of the other factors of the extrinsic pathway (factor II, factor V, and factor X), as it is directly activated by exposed TF and directly activates factor X in turn, initiating the extrinsic coagulation cascade.

It would be appreciated by one skilled in the art, that after reduction of the ionic strength of the composition according to some embodiments of the present invention to near-physiological levels and in the presence of calcium buffers such as citrate or oxalate or EDTA or others, this composition is expected to be stable for at least 24 hours and up to a period of approximately 30 days at temperatures of about 2-25°C. The composition may be frozen and stored for an even longer period (up to six months). The addition of stabilizers as described hereinabove would significantly extend these time periods. Innocuous cryopreservatives (e.g. mannitol, sucrose, PEG, and others known in the art) may also be added to improve the recovery of coagulation factor activity after freeze/thaw. Therefore, the composition disclosed herein may be prepared for a patient either immediately or in advance in preparation for surgery.

In some embodiments, prior to application, the composition according to some embodiments of the present invention may be pre-activated with a calcium containing solution. In some embodiments, in order to begin the activation cascade of the clotting factors and in order to facilitate both the activation of platelets and the release of factor V and growth factors typically found in platelets (in those embodiments where platelets may be retained), it may be advantageous to add a calcium containing solution (e.g. CaCl₂) to a final concentration of about 5-100mM. In some embodiments, the final concentration of CaCl₂ is about 10-80mM. In some embodiments, the final concentration of CaCh is about 20-40mM. This solution may either be added to the elution buffer, or to the equilibration buffer, or to the composition prior to application.

In some embodiments, a composition for preventing or reducing tissue adhesions can include one or more of the following properties: forms a barrier limited to the tissue at the surgical site per se. This barrier may be composed of endogenous Fibrinogen which is activated by the extract to polymerize in such a way that is not permissive to adhesion forming cells; is hemostatic and thus reduces fibrin deposition; does not leave significant levels of extant fibrin such as is associated with fibrin sealant at the surgical site or wound site (e.g. due to the reduction of Fibrinogen); and can be made at point of care or at a nearby facility (e.g. the hospital's blood bank). The composition of the present invention provides such a barrier as the compositions according to some embodiments of the present invention.

In some embodiments, the composition according to some embodiments of the present invention comprises endogenous microvesicles and phospholipids. In some embodiments, the composition according to some embodiments of the present invention comprises factor V, endogenous microvesicles, phospholipids, or any combination thereof. In some embodiments, the composition according to some embodiments of the present invention comprises platelets. In some embodiments, the composition according to some embodiments of the present invention is substantially depleted of red blood cells (RBC) and white blood cells (WBC), and includes less than about 1% RBC and/or 1% WBC. In some embodiments, the composition according to some embodiments of the present invention has less than about 0.5% RBC and/or 0.5% WBC.

### A method for generating a composition according to some embodiments of the present invention (the methods described below in this section of the disclosure are not covered by the claimed invention)

The present disclosure provides a method to obtain the composition according to some embodiments of the present invention, comprising:
obtaining a blood plasma sample,
producing a bound fraction by applying the blood plasma sample to an anion exchange column, and
obtaining a composition according to some embodiments of the present invention by eluting the bound fraction with an elution solution.

In some embodiments, the blood plasma sample is obtained from a source selected from the group consisting of: a subject undergoing surgery, a donor, and a commercially available source. In some embodiments, the blood plasma sample is between 5mL to 500mL.

In some embodiments, the composition comprises proaccelerin (factor V), and at least one factor selected from the group consisting of: prothrombin (factor II), proconvertin (factor VII), and Stuart-Prower factor (factor X),
wherein an amount of the proaccelerin in the composition is between 75% to 750% compared to an amount of proaccelerin in a blood plasma, and
wherein an amount of the at least one factor is from 150% to 3000% compared to an amount of the at least one factor in the blood plasma;
wherein the amount of proaccelerin and the at least one factor in the composition is determined by extrapolating an observed activity of the composition at a concentration of 500mM NaCl, using a prothrombin complementation assay using a standard curve constructed using the blood plasma.

In some embodiments, the composition further comprises fibrin stabilizing factor (factor XIII), wherein the concentration of the fibrin stabilizing factor in the composition is between 0.01 IU/mL to 100 IU/mL.

In some embodiments, the composition obtained by eluting from the anion exchange column is a plasma derived concentrate.

In some embodiments, the composition obtained by eluting from the anion exchange column is a fibrinogen-depleted plasma-derived concentrate.

In some embodiments, the method is performed under sterile conditions.

In some embodiments, the anion exchange column is sterile.

In some embodiments, the elution solution is sterile.

In some embodiments, the method further comprises washing the bound fraction with a wash solution prior to eluting the bound fraction with the elution solution.

In some embodiments, the wash solution is sterile.

In some embodiments, the blood plasma sample is obtained from a source selected from the group consisting of: a subject undergoing surgery, a donor, and a commercially available source.

In some embodiments, the blood plasma sample is between 5mL to 500mL.

In some embodiments, the anion exchange column has a bed volume range from 0.5 mL to 20 mL.

In an embodiment, the anion exchange column has a bed volume range from 1 mL to 20 mL. In an embodiment, the anion exchange column has a bed volume range from 5 mL to 20 mL. In an embodiment, the anion exchange column has a bed volume range from 10 mL to 20 mL. In an embodiment, the anion exchange column has a bed volume range from 15 mL to 20 mL. In an embodiment, the anion exchange column has a bed volume range from 0.5 mL to 15 mL. In an embodiment, the anion exchange column has a bed volume range from 0.5 mL to 10 mL. In an embodiment, the anion exchange column has a bed volume range from 0.5 mL to 5 mL.

In an embodiment, the anion exchange column has a bed volume range from 0.5 mL to 5 mL. In an embodiment, the anion exchange column has a bed volume range from 1 mL to 5 mL. In an embodiment, the anion exchange column has a bed volume range from 2 mL to 5 mL. In an embodiment, the anion exchange column has a bed volume range from 3 mL to 5 mL. In an embodiment, the anion exchange column has a bed volume range from 4 mL to 5 mL. In an embodiment, the anion exchange column has a bed volume range from 0.5 mL to 4 mL. In an embodiment, the anion exchange column has a bed volume range from 0.5 mL to 3 mL. In an embodiment, the anion exchange column has a bed volume range from 0.5 mL to 2 mL. In an embodiment, the anion exchange column has a bed volume range from 0.5 mL to 1 mL. In an embodiment, the anion exchange column has a bed volume range from 1 mL to 4 mL. In an embodiment, the anion exchange column has a bed volume range from 2 mL to 3 mL.

In some embodiments, an amount of resin of the anion exchange column is between 1:1 to 50:1 blood plasma sample:resin. In an embodiment, an amount of resin of the anion exchange column is between 5:1 to 50:1 blood plasma sample:resin. In an embodiment, an amount of resin of the anion exchange column is between 10:1 to 50:1 blood plasma sample:resin. In an embodiment, an amount of resin of the anion exchange column is between 15:1 to 50:1 blood plasma sample:resin. In an embodiment, an amount of resin of the anion exchange column is between 20:1 to 50:1 blood plasma sample:resin. In an embodiment, an amount of resin of the anion exchange column is between 25:1 to 50:1 blood plasma sample:resin. In an embodiment, an amount of resin of the anion exchange column is between 30:1 to 50:1 blood plasma sample:resin. In an embodiment, an amount of resin of the anion exchange column is between 35:1 to 50:1 blood plasma sample:resin. In an embodiment, an amount of resin of the anion exchange column is between 40:1 to 50:1 blood plasma sample:resin. In an embodiment, an amount of resin of the anion exchange column is between 45:1 to 50:1 blood plasma sample:resin.

In some embodiments, the blood plasma sample comprises between 1 to 50 column volumes. In some embodiments, the blood plasma sample comprises between 1 to 40 column volumes. In some embodiments, the blood plasma sample comprises between 1 to 30 column volumes. In some embodiments, the blood plasma sample comprises between 1 to 20 column volumes. In some embodiments, the blood plasma sample comprises between 1 to 10 column volumes. In some embodiments, the blood plasma sample comprises between 10 to 50 column volumes. In some embodiments, the blood plasma sample comprises between 20 to 50 column volumes. In some embodiments, the blood plasma sample comprises between 30 to 50 column volumes. In some embodiments, the blood plasma sample comprises between 40 to 50 column volumes. In some embodiments, the blood plasma sample comprises between 10 to 40 column volumes. In some embodiments, the blood plasma sample comprises between 20 to 30 column volumes.

In some embodiments, the eluting comprises contacting the bound fraction with 0.5 to 10 column volumes of high salt buffer. In some embodiments, the eluting comprises contacting the bound fraction with 1 to 10 column volumes of high salt buffer. In some embodiments, the eluting comprises contacting the bound fraction with 2 to 10 column volumes of high salt buffer. In some embodiments, the eluting comprises contacting the bound fraction with 3 to 10 column volumes of high salt buffer. In some embodiments, the eluting comprises contacting the bound fraction with 4 to 10 column volumes of high salt buffer. In some embodiments, the eluting comprises contacting the bound fraction with 5 to 10 column volumes of high salt buffer. In some embodiments, the eluting comprises contacting the bound fraction with 6 to 10 column volumes of high salt buffer. In some embodiments, the eluting comprises contacting the bound fraction with 7 to 10 column volumes of high salt buffer. In some embodiments, the eluting comprises contacting the bound fraction with 8 to 10 column volumes of high salt buffer. In some embodiments, the eluting comprises contacting the bound fraction with 9 to 10 column volumes of high salt buffer.

In some embodiments, the eluting comprises contacting the bound fraction with 0.5 to 9 column volumes of high salt buffer. In some embodiments, the eluting comprises contacting the bound fraction with 0.5 to 8 column volumes of high salt buffer. In some embodiments, the eluting comprises contacting the bound fraction with 0.5 to 7 column volumes of high salt buffer. In some embodiments, the eluting comprises contacting the bound fraction with 0.5 to 6 column volumes of high salt buffer. In some embodiments, the eluting comprises contacting the bound fraction with 0.5 to 5 column volumes of high salt buffer. In some embodiments, the eluting comprises contacting the bound fraction with 0.5 to 4 column volumes of high salt buffer. In some embodiments, the eluting comprises contacting the bound fraction with 0.5 to 3 column volumes of high salt buffer. In some embodiments, the eluting comprises contacting the bound fraction with 0.5 to 2 column volumes of high salt buffer. In some embodiments, the eluting comprises contacting the bound fraction with 0.5 to 1 column volumes of high salt buffer. In some embodiments, the eluting comprises contacting the bound fraction with 1 to 9 column volumes of high salt buffer. In some embodiments, the eluting comprises contacting the bound fraction with 2 to 8 column volumes of high salt buffer. In some embodiments, the eluting comprises contacting the bound fraction with 3 to 7 column volumes of high salt buffer. In some embodiments, the eluting comprises contacting the bound fraction with 4 to 6 column volumes of high salt buffer.

In some embodiments, the high salt buffer comprises a salt solution (e.g.,but not limited to, NaCl, KCl, etc.) between 500 mM and 1000 mM. In some embodiments, the salt solution is between 500 and 1000 mM NaCl (e.g., but not limited to, 500mM NaCl, 750mM NaCl, 1000mM NaCl, etc.).

In some embodiments, the method optionally comprises a wash step, wherein the bound fraction is washed with 0.3 to 20 column volumes of low salt solution. In some embodiments, the low salt solution is a salt solution between 0 to 200mM (e.g., but not limited to, NaCl, KCl, etc.). In some embodiments, the low salt solution is between 0 and 100 mM NaCl (e.g., but not limited to, 0mM NaCl, 25mM NaCl, 50mM NaCl, 75mM NaCl, etc.).

In some embodiments, the bound fraction is washed with 1 to 20 column volumes of low salt solution. In some embodiments, the bound fraction is washed with 5 to 20 column volumes of low salt solution. In some embodiments, the bound fraction is washed with 10 to 20 column volumes of low salt solution. In some embodiments, the bound fraction is washed with 15 to 20 column volumes of low salt solution. In some embodiments, the bound fraction is washed with 1 to 15 column volumes of low salt solution. In some embodiments, the bound fraction is washed with 1 to 10 column volumes of low salt solution. In some embodiments, the bound fraction is washed with 1 to 5 column volumes of low salt solution. In some embodiments, the bound fraction is washed with 5 to 15 column volumes of low salt solution. In some embodiments, the bound fraction is washed with 5 to 10 column volumes of low salt solution. In some embodiments, the bound fraction is washed with 10 to 15 column volumes of low salt solution.

In some embodiments, the column is washed from weakly binding or non-specific proteins in the same orientation as the previous (loading) step. A buffered or unbuffered saline solution of about 0-500 mM ionic strength, typically about 50-250 mM, can therefore be used to wash the column by connecting a wash-solution containing syringe, and depressing the plunger to push the solution through the column, or utilizing an automated solution. The orientation of the wash is typically the same as that of the plasma. The buffer is typically a citrate or oxalate-based buffer, but other physiological buffers (e.g. phosphate, carbonate, acetate) may be used. The pH is typically about pH 5.0-8.5, about pH 6-8, or about pH 6.5-7.5.

In some embodiments, where autologous or single-donor (e.g. a family member or other donor) plasma is used, the first step is to collect blood and separate the plasma fraction. Blood from the subject having the surgery, or from the donor, is collected in a vessel containing an anticoagulant solution. For example, this is a citrated vacutainer (e.g., a standard blood collection tube), or a standard collection bag containing a citrate buffer such as citrate phosphate dextrose adenine (CPD-A). The citrate-containing solution in the vacutainer may also include other excipients such as those typically used for platelet rich plasma collection (e.g. anticoagulant citrate dextrose solution, solution A ("ACD-A") or anticoagulant citrate dextrose solution, solution B ("ACD-B"), also containing dextrose and adenosine; supplied by, e.g., but not limited to, Citra Labs). Other anticoagulant solutions may also be used (e.g., but not limited to, other citrate-based solutions such as CPD-A or others, oxalate-based solutions, EDTA-solutions, heparin solutions, heparinized vacutainers, etc.). It is assumed that the final concentration of the composition according to some embodiments the present invention should be about 2 to 10 fold over the concentration of the components in plasma, e.g., but not limited to, about 4 to 6 fold. As approximately 50% of the blood volume is composed of the cellular fraction, mostly red blood cells, a volume of blood of at least 4 fold and at most 20 fold over the final volume, would be collected, respectively. Correspondingly, for preparing approximately 10 to 20 ml of the composition according to some embodiments of the present invention, about 100 to 200 ml of blood would therefore typically be collected. This volume may be collected in any number and size of vacutainers or vessels as per the practitioner's convenience. Standard equipment for aseptic blood collection may be used for the collection of the blood, and many blood collection sets, bags, and vacutainer types are commercially available for this purpose.

In some embodiments of the method, composition according to some embodiments of the present invention originates from autologous blood. The use of autologous plasma removes the risks associated with the use of commercially available blood-based products. In many situations, whether due to the possibility where a surgical subject is unable to give blood, or where the hospital is not equipped to collect and maintain autologous blood or plasma, a unit of plasma from a single donor that has been screened for pathogens, may also be used. Another potential safe source for plasma is commercially available viral-inactivated human plasma. The blood or plasma source does not require typing, however, as few or no red blood cells containing the A, B, or Rh antigens, or white blood cells, are found in composition according to some embodiments of the present invention. This allows for the use of non-compatible blood types so that any individual may donate blood, e.g. a family member or a third party who has been screened for dangerous pathogens.

Autologous blood-derived products have an additional advantage of nullifying any risk of transmissible disease (primarily blood-borne viruses and/or prion diseases). Optionally, in some embodiments of the method of the present disclosure, a screened blood donation from a single donor may be used, for example any standard unit of plasma for infusion in a hospital, lyophilized plasma (LyoplasN, DRK-Blutspendedienst West). To reduce potential risks associated with allogeneic blood, donor plasma can be treated with non-ionic solvent-detergent for viral removal (e.g. Triton X-100, Tween-80, TNBP and others of similar nature) and used using the described method and kit. Commercially available pooled plasma pre-treated with solvent-detergent or which has undergone any other type of viral inactivation procedure known in the art (e.g. nanofiltration or UV-treatment) may also be utilized (e.g., but not limited to, Octaplas^{®}, Octapharma).

In some embodiments, allogeneic plasma (e.g., plasma derived from a compatible donor into the subject) is used. Allogeneic plasma is a blood product which can be obtained from, but is not limited to, blood donations.

In some embodiments, as plasma is stable for several days under refrigeration and months if frozen, it is also possible to perform autologous blood collection prior to surgery, for example in cases where surgery is scheduled in advance (e.g. Caesarian surgery in the case of a confirmed pregnancy).

In some embodiments, the plasma may be stored for up to about 24 hours. In some embodiments, the plasma may be stored for up to about six days at approximately 4° Celsius (e.g. in a standard refrigerator). In some embodiments, the plasma may be stored for up to three-six months frozen (e.g. in a standard freezer reaching a freezing temperature of approximately -18° to -30° Celsius). In some embodiments, the plasma is allogeneic of any of the sources named above (e.g., but not limited to, an anonymous donor, a commercial virally inactivated plasma, or any combination thereof).

In some embodiments, in order to reduce the stress on the patient or donor, it is also possible to withdraw the blood plasma while returning the red blood cells to the body, e.g., but not limited to, using plasmapheresis. Such techniques are well known in the art and are the standard for generating plasma for commercial purposes (see, e.g., information on plasma collection in Octapharma: http://octapharmaplasma.com/donor/).

In some embodiments, blood collection can therefore be performed either in the point-of-care (e.g. the operating room (OR)), or in a separate facility (e.g. the hospital blood bank or plasma collection center) that is equipped with the necessary standard equipment for phlebotomy and plasma separation.

In cases where an anonymous donation (e.g., but not limited to, blood bank plasma) or commercial viral inactivated plasma (e.g., but not limited to, Octaplas^{®}) is used, in some embodiments, the first step consists of thawing the plasma using standard techniques.

Optionally, in some embodiments where non-autologous plasma has not been virally inactivated, solvent-detergent treatment (SD-treatment) can be performed. This consists of mixing within the plasma a volume of 0.5 to 3% v/v of a non-ionic solvent-detergent. Non-limiting examples of such materials are Tween-80, NP-40, Triton X-100 (TX-100), and others. The solvent-detergent and plasma are thoroughly mixed and allowed to incubate for at least 30 minutes to dissolve viral membranes. In the case that SD-treatment is performed, an additional wash step is needed after loading the anion-exchanger to remove the solvent-detergent. In some embodiments, an additional wash step of at least 5 column volumes, e.g., at least 10 column volumes, with a buffered solution either containing or not containing saline is performed on the SD-treated plasma prior to the wash step using a low salt solution.

In some embodiments fibrinogen depletion is performed by optimizing the wash and elution buffers so as to remove a majority of the relatively weakly bound fibrinogen (as compared to other coagulation factors) from the anion exchange column or other type of manifold while retaining other coagulation factors. In some embodiments, the plasma is depleted of fibrinogen by precipitation out of the plasma using a biocompatible polymer or mixture of biocompatible polymers prior to loading the plasma onto the anion exchange column or other type of manifold containing an anion exchange matrix. In some embodiments, removing fibrinogen is achieved by a wash step during the anion exchange purification of the extract. Fibrinogen-depleted plasma refers to blood plasma containing no more than about 0.3 g/liter, about 0. 1g/liter or about 0.05 g/liter fibrinogen.

Optionally, in some embodiments, to deplete fibrinogen from the plasma in order to generate the composition according to some embodiments of the present invention, a polymeric solution or solid powder of a biocompatible polymer may be added using a syringe into the vacutainer, or may be provided within a collection bag/vessel (e.g., but not limited to, a standard plasma bag provided in a standard collection bag/vessel which contains citrate buffer (e.g., but not limited to, CPD-A). In some embodiments, an anion exchange (AEX) column is used for the removal of fibrinogen to the composition according to some embodiments of the present invention. In some embodiments, polymers such as polyethylene glycol (PEG) can been used in defibrinylating plasma (e.g., Li et al. Thromb Res. 1995 79(4):395-403.). Other polymers (dextran, hyaluronic acid, chondroitin sulfate, poly-lysine, or any other biologically compatible polymer (Iverius PH and Laurent TC. Biochim Biophys Acta. 1967 133(2):371-3), may also be used for defibrinylating plasma. The addition of a polymer may also reduce red blood cell (RBC) lysis (Kameneva, et al., ASAIO J. 2003 49(5):537-42.). Due to the wide variety in available biocompatible polymers (e.g., but not limited to, dextran and PEG) and biopolymers (polymers of a biological origin, e.g. heparin, hyaluronic acid, and chondroitin sulfate), and available molecular weight ranges, this step may require optimization depending on the polymer selected. In some embodiments, the tube also contains a high molecular weight biocompatible polymer to facilitate fibrinogen precipitation. In some embodiments, a biocompatible polymer is added to the tube. In some embodiments, the biocompatible polymer is selected from PEG, dextran, chondroitin sulfate, heparin, or hyaluronic acid. The utilization of vacutainers, which are provided sterile by many manufacturers and maintain an inherent level of negative pressure (vacuum) within, allows the easy addition of a small amount of polymer solution by injecting it through the rubber cap or stopper. Other techniques may be used at the convenience of the practitioner (e.g., but not limited to, a physician).

The composition described here is produced from mammalian blood (human blood for clinical applications or animal blood, e.g. for veterinary applications), e.g., autologous blood, or blood from donors that has been screened for pathogens. Also, no equipment coming in touch with plasma is reused and therefore no toxic substances or buffers are required (e.g. NaOH typically used for column cleaning between runs). Furthermore, the method does not require a complete purification and separation of the clotting factors from other blood proteins and components. One main advantage of the method described herein, therefore, is that the relative and absolute concentration of the clotting factors needs to be increased but only a partial purification is required. This is in stark contrast to any known standard plasma-derived commercial product.

In some embodiments, the present method removes processing requirements such as constant and controlled flow, monitoring of various parameters such as flow rate, pressure, UV absorbance, pH, conductivity, and the like, thus eliminating the need for dedicated equipment and personnel to maintain and monitor these parameters. A further development of the method described here regards the utilization of entry and exit filters on the column. It would be appreciated that it may be advantageous to retain small particles (e.g. microvesicles, platelet fragments, and intact platelets that are typically 3-5 um wide) as these facilitate the clotting of endogenous blood, carry high levels of factor V, and are a source of phospholipids for the assembly of the coagulation factor complex. It is, however, advantageous to remove larger components such as red blood cells, and immune (white blood) cells which could block anion exchange columns, undergo lysis thus releasing inflammatory molecules or otherwise potentiate inflammation, as these may be inadvertently aspired during plasma collection.

The step of separating the plasma from the red and white blood cells may be carried out using methods known in the art. In some embodiments, the separation is performed by centrifugation. In some embodiments an inert gel composition is used to separate the plasma and platelets from the red and white blood cells, such as is known in the art for platelet rich plasma (PRP) separation (non limiting examples of commercially products include the RegenKit^{®} THT [e.g., http://sportsmedicine.stryker.com/Product/RegenKit-THT/73/RegenKit-THT-Autologous-Platelet-Rich-Plasma-Brochure; PLATELET GelSep^{®} http://www.cabru.com/public/001BC7006T.pdf]; and others).

In some embodiments, the separation is performed by centrifugation whereby a biocompatible polymer (e.g. polymer useful for precipitating fibrinogen from the blood) is added to the blood prior to centrifugation in order to yield fibrinogen-depleted plasma. The biocompatible polymer may be added to the blood at collection or post-collection. The fibrinogen-depleted plasma may be prepared by, for example, centrifuging the blood. In such a manner, a composition according to some embodiments of the present invention that is at least partially depleted of fibrinogen and RBC and WBC may be obtained.

In some embodiments, the method of the present disclosure includes loading of the AEX column with the plasma, in the desired orientation regarding the retention of particles as described hereinabove. This step is performed by attaching the syringe containing the plasma to the column, either directly or via an adapter. In some embodiments, the syringe is attached via a luer-lock mechanism, via a flexible tube or via any other mechanism.

In some embodiments, the plunger of the syringe is depressed to push the plasma through the anion exchange column. The main coagulation factors of the Extrinsic System (e.g., factors II, VII, and X) and factors associated thereto (e.g. factor V) are strongly bound to the column via their gla-domain interaction with the anion exchange functional groups on the resin inside, or via their interaction with the gla-domain bearing proteins.

In some embodiments, in the next step, coagulation factors and associated factors with or without the microvesicles, platelets and fragments thereof, and phospholipids, are eluted from the column. In one embodiment the eluate (composition according to some embodiments of the present invention) is collected in an eluate collection container, such as a bottle, syringe or the like. In some embodiments, the eluate collection container contains a diluent, e.g., to reduce the elution saline concentration (e.g., but not limited to, water and/or any pharmaceutically acceptable buffer or solution). The elution of the composition according to some embodiments of the invention is achieved by eluting using an elution buffer which includes a high salt concentration. Without being bound to theory, the negatively charged salt ions compete with the negative charges on the bound molecules. Typical salts and concentrations are, for example, citrate-buffered solutions containing about 100-2000 mM NaCl, or about 250-1000mM, or about 500-800mM. A calcium salt, for example CaCh at about 5-100mM, or about 10-40mM, may be added to the elution buffer. Without being bound to theory, some coagulation factors are activated by free calcium ions. In addition, platelets are activated by the calcium ions thus increasing the available surface for interaction and releasing coagulation factors such as factor V, as an additional source of factor V.

In some embodiments, the elution can be performed in several methods for example into a container or into a second syringe that is mounted on the other side of the column in the depressed position. The pushing of the plunger of the elution syringe would force the elution solution through the column and into the second syringe. The solution may be returned through the column to the elution syringe, and back, in order to expose the column to a high ionic concentration several times, thereby increasing the efficiency of elution. The container can also be any other suitable container, for example a sterile spray bottle.

In some embodiments, the high salt in the elution buffer is adjusted. Physiological blood salt concentrations are equivalent to approximately 290-320 mOsm, which is provided by a concentration of about 145-160 mM of NaCl. In some embodiments, the salt concentration is adjusted to about 100 to 750 mM. In some embodiments, the salt concentration is adjusted to about 150 to 350mM. In some embodiments, the salt concentration is adjusted to about 150 to 275 mM. Reducing the salt concentration is preferable to avoid a final product that is hypertonic, and to reduce the degradation of the salt-labile factor V and/or other salt-labile factors. In addition, the adjustment of the final concentration ratio of the extract is determined in this step. The salt adjustment may be performed by the addition of water, or buffer solution without salts or with a low salt concentration, or by performing the elution step into a container such as a spray bottle, syringe, or other container, already containing a pre-measured amount of water or buffer.

The disadvantages and dangers of the known sealants, clotting factor concentrates and barriers are multiple.

The production of a PPSB fraction (an acronym for: Prothrombin [Factor II], Proconvertin [Factor VII], Stuart factor [Factor X], and anti-hemophilia factor B [Factor IX]) for commercial purposes has been described as an intermediate in the production of other plasma proteins (e.g. thrombin), or as an antidote for anti-coagulation therapy (in the form of Prothrombin Complex Concentrate ("PCC")). The methods, however, relate primarily to the isolation of these four factors. Additional factors that are normally associated with the PPSB factors in the blood, for example pro-accelerin (Factor V, FV), and phospholipids, are not expected to persist throughout the production processes known in the industry. Factor VII (FVII) is another labile factor typically degraded in commercial PCCs. PCCs are typically described as 3-factor of 4-factor PCCs depending on the lack, or presence (in variable amounts) of factor VII. PCC concentrations are correspondingly generally calculated based on the relative concentration of the more stable Factor IX (FIX).

Additionally, the commercial production of the PPSB fraction relies on numerous pieces of machinery and a lengthy and complex process (e.g. storage tanks for buffers and plasma, large columns, specialized centrifuges e.g. continuous flow centrifuges, chromatography machinery, various monitors and gauges, lyophilization, filling machines, etc.), a large number of dedicated personnel, and the processing of large volumes of blood from a large pool of donors to generate a plasma pool. These are a consequence of the processing volumes and requirements for a viable commercial process, geared and designed to generate many doses of medicaments. In contrast, the process described herein is performed per patient, from a small volume of plasma (typically a single plasma unit weighing less than 0.5 kilogram), and is therefore able to be performed in the point of care or nearby, rapidly - within the timeframe of a single operation, and on demand - requiring no more than a single operator to perform.

An additional complication in the generation of blood products such as PCCs, is the dependence on pooled plasma from a large number of allogenic blood donors. This mandates two viral removal steps, a requirement demanded by most regulatory authorities (e.g., but not limited to, EMA Guideline on plasma-derived medicinal products, section 8.1.1. http://www.ema.europa.eu/docs/en_GB/document_library/Scientific_guideline/2011/07/ WC500109627.pdf), requiring further time and processing. Therefore, the minimal time required for processing is between several hours to days. Thus, a fundamental difference between existing processes and the process described herein is the applicability of performing this process for a single patient, in the point of care (e.g. but not limited to the operating room), in the timeframe required for use during an operation.

In addition, most viral inactivation steps used in the industry, such as solvent-detergent treatment or nanofiltration, will entirely remove lipid components (e.g. microparticles, platelets and platelet fragments, and phospholipids).

Another disadvantage of known methods is that for storage and transportation, the products have to be stabilized, typically by the addition of excipients and stabilizers, and typically a lengthy and costly freeze-drying procedure, further requiring additional specialized equipment and facilities. The use of a process performed on demand, and yielding an extract that can be used immediately, precludes this step.

Accordingly, in some embodiments, the present disclosure is a method for the rapid production of small amounts of coagulation factors including both the PPSB and the associated cofactors (e.g. factor V, phospholipids etc.) from minimal volumes (5ml to 500 ml) of whole blood without utilizing cumbersome equipment (e.g. storage tanks, pumps, various monitors, buffer reservoirs, continuous centrifuges, filling lines, etc.).

It would be appreciated that the directionality of the flow during the different procedures detailed herein (loading, washing, and eluting from the column) has significance regarding the type of particles that are retained during the process.

In some embodiments, the composition of the present invention may have a reduced viral and/or bacterial population not previously detected in the plasma screens performed routinely in a hospital setting.

### A composition according to some embodiments of the present invention for use in reducing adhesion severity

In another aspect, the present invention provides the composition according to the other aspect of the present invention for use in reducing adhesion severity by topical application at a surgical site in a subject undergoing surgery, wherein the composition is an aqueous composition.

In some embodiments, the composition for use of the present invention is diluted between 1 and 5 times, e.g., but not limited to, 1:1 dilution, 1:2 dilution, 1:3 dilution, 1:4 dilution, or 1:5 dilution, prior to administration. The dilution can be in a physiological solution, such as, for example, saline.

In some embodiments, the composition for use according to some embodiments of the present invention is for use in reducing adhesion severity by topical application at a surgical site of the subject undergoing surgery by at least 10% compared to an adhesion severity at a surgical site in an untreated subject undergoing surgery, wherein the composition is an aqueous composition. In some embodiments, the composition for use according to some embodiments of the present invention is for use in reducing adhesion severity by topical application at a surgical site of the subject undergoing surgery by at least 15% compared to an adhesion severity at a surgical site in an untreated subject undergoing surgery, wherein the composition is an aqueous composition. In some embodiments, the composition for use according to some embodiments of the present invention is is for use in reducing adhesion severity by topical application at a surgical site of the subject undergoing surgery by at least 20% compared to an adhesion severity at a surgical site in an untreated subject undergoing surgery, wherein the composition is an aqueous composition. In some embodiments, the composition for use according to some embodiments of the present invention is for use in reducing adhesion severity by topical application at a surgical site of the subject undergoing surgery by at least 25% compared to an adhesion severity at a surgical site in an untreated subject undergoing surgery, wherein the composition is an aqueous composition.

In some embodiments, the composition for use according to some embodiments of the present invention is for use in reducing adhesion severity by topical application at a surgical site of the subject undergoing surgery in an amount sufficient to reduce an adhesion severity at the surgical site by 10% to 25%compared to an adhesion severity at a surgical site in an untreated subject undergoing surgery, wherein the composition is an aqueous composition. In some embodiments, the composition for use according to some embodiments of the present invention is for use in reducing adhesion severity by topical application at a surgical site of the subject undergoing surgery in an amount sufficient to reduce an adhesion severity at the surgical site by 15% to 25% compared to an adhesion severity at a surgical site in an untreated subject undergoing surgery, wherein the composition is an aqueous composition. In some embodiments, the composition for use according to some embodiments of the present invention is for use in reducing adhesion severity by topical application at a surgical site of the subject undergoing surgery by 20% to 25% compared to an adhesion severity at a surgical site in an untreated subject undergoing surgery, wherein the composition is an aqueous composition.

In some embodiments, the composition for use according to some embodiments of the present invention, is for use in reducing adhesion severity by topical application at a surgical site of the subject undergoing surgery by 10% to 20% compared to an adhesion severity at a surgical site in an untreated subject undergoing surgery, wherein the composition is an aqueous composition. In some embodiments, the composition for use according to some embodiments of the present invention is for use in reducing adhesion severity by topical application at a surgical site of the subject undergoing surgery by 10% to 15% compared to an adhesion severity at a surgical site in an untreated subject undergoing surgery, wherein the composition is an aqueous composition. In some embodiments, the composition for use according to some embodiments of the present invention is for use in reducing adhesion severity by topical application at a surgical site of the subject undergoing surgery by 15% to 20% compared to an adhesion severity at a surgical site in an untreated subject undergoing surgery, wherein the composition is an aqueous composition.

In some embodiments, the composition for use according to some embodiments of the present invention is for use to reduce an amount of adhesions (i.e., a number of adhesions) by topical application at a surgical site of the subject undergoing surgery by at least 10%compared to an amount of adhesions at a surgical site in an untreated subject undergoing surgery, wherein the composition is an aqueous composition. In some embodiments, the composition for use according to some embodiments of the present invention is for use to reduce an amount of adhesions (i.e., a number of adhesions) by topical application at a surgical site of the subject undergoing surgery by at least 15%, compared to an amount of adhesions at a surgical site in an untreated subject undergoing surgery, wherein the composition is an aqueous composition. In some embodiments, the composition for use according to some embodiments of the present invention is for use to reduce an amount of adhesions (i.e., a number of adhesions) by topical application at a surgical site of the subject undergoing surgery by at least 20%, compared to an amount of adhesions at a surgical site in an untreated subject undergoing surgery, wherein the composition is an aqueous composition. In some embodiments, the composition for use according to some embodiments of the present invention is for use to reduce an amount of adhesions (i.e., a number of adhesions) by topical application at a surgical site of the subject undergoing surgery by at least 25%, compared to an amount of adhesions at a surgical site in an untreated subject undergoing surgery, wherein the composition is an aqueous composition.

In some embodiments, the composition for use according to some embodiments of the present invention is for use to reduce an amount of adhesions (i.e., a number of adhesions) by topical application at a surgical site of the subject undergoing surgery by 10% to 25%, compared to an amount of adhesions at a surgical site in an untreated subject undergoing surgery, wherein the composition is an aqueous composition. In some embodiments, the composition for use according to some embodiments of the present invention is for use to reduce an amount of adhesions (i.e., a number of adhesions) by topical application at a surgical site of the subject undergoing surgery by 15% to 25%, compared to an amount of adhesions at a surgical site in an untreated subject undergoing surgery, wherein the composition is an aqueous composition. In some embodiments, the composition for use according to some embodiments of the present invention is a for use to reduce an amount of adhesions (i.e., a number of adhesions) by topical application at a surgical site of the subject undergoing surgery by 20% to 25%, compared to an amount of adhesions at a surgical site in an untreated subject undergoing surgery, wherein the composition is an aqueous composition.

In some embodiments, the composition for use according to some embodiments of the present invention is for use to reduce an amount of adhesions (i.e., a number of adhesions) by topical application at a surgical site of the subject undergoing surgery by 10% to 20%, compared to an amount of adhesions at a surgical site in an untreated subject undergoing surgery, wherein the composition is an aqueous composition. In some embodiments, the composition for use according to some embodiments of the present invention is for use to reduce an amount of adhesions (i.e., a number of adhesions) by topical application at a surgical site of the subject undergoing surgery by 10% to 15% compared to an amount of adhesions at a surgical site in an untreated subject undergoing surgery, wherein the composition is an aqueous composition. In some embodiments, the composition for use according to some embodiments of the present invention is for use to reduce an amount of adhesions (i.e., a number of adhesions) by topical application at a surgical site of the subject undergoing surgery by 15% to 20% compared to an amount of adhesions at a surgical site in an untreated subject undergoing surgery, wherein the composition is an aqueous composition.

In some embodiments, the composition for use according to some embodiments of the present invention,is for use to reduce an adhesion area by topical application at a surgical site of the subject undergoing surgery by at least 10%, compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery by at least 15% compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery by at least 20% compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery in by at least 25% compared to an adhesion area at a surgical site in an untreated subject undergoing surgery, wherein the composition is an aqueous composition.

In some embodiments, the composition for use according to some embodiments of the present invention is for use to reduce an adhesion area by topical application at a surgical site of the subject undergoing surgery at the surgical site by 10% to 25%compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery in an amount sufficient to reduce an adhesion area at the surgical site by 15% to 25% compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery in an amount sufficient to reduce an adhesion area at the surgical site by 20% to 25% compared to an adhesion area at a surgical site in an untreated subject undergoing surgery, wherein the composition is an aqueous composition.

In some embodiments, the composition for use according to some embodiments of the present invention is for use to reduce an adhesion area by topical application at a surgical site of the subject undergoing surgery at the surgical site by 10% to 20%compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery by 10% to 15% compared to an adhesion area at a surgical site in an untreated subject undergoing surgery, wherein the composition is an aqueous composition. In some embodiments, the composition for use according to some embodiments of the present invention is for use to reduce an adhesion area by topical application at a surgical site of the subject undergoing surgery by 15% to 20% compared to an adhesion area at a surgical site in an untreated subject undergoing surgery, wherein the composition is an aqueous composition.

The composition for use comprises proaccelerin (factor V) and prothrombin (factor II), proconvertin (factor VII), and Stuart-Prower factor (factor X),
wherein the concentration of proaccelerin is from 300% to 2000% relative to the concentration in a normal blood plasma sample, wherein the concentration is determined by extrapolating the activity of proaccelerin using a clotting assay and plotting a standard curve;
wherein the concentration of each one of proconvertin, prothrombin and Stuart-Prower factor is from 500% to 2500% or from 150% to 2000% relative to the concentration in a normal blood plasma sample; and
wherein the composition is a fibrinogen-depleted plasma-derived concentrate.
wherein an amount of the proaccelerin in the composition is between 75% to 750% compared to an amount of proaccelerin in a blood plasma, and
wherein an amount of the at least one factor is from 150% to 3000% compared to an amount of the at least one factor in the blood plasma;
wherein the amount of proaccelerin and the at least one factor in the composition is determined by extrapolating an observed activity of the composition at a concentration of 500mM NaCl, using a prothrombin complementation assay using a standard curve constructed using the blood plasma.

In some embodiments, an amount of fibrinogen in the composition is reduced by 5% to 95% of an amount of fibrinogen in the blood plasma. In some embodiments, an amount of fibrinogen in the composition is reduced by 25% to 75% of an amount of fibrinogen in the blood plasma. In some embodiments, the amount of fibrinogen in the composition is tested using a Clauss assay.

In some embodiments, the composition further comprises fibrin stabilizing factor (factor XIII), wherein the concentration of the fibrin stabilizing factor in the composition is between 0.01 IU/mL to 100 IU/mL.

In some embodiments, the severity of adhesions is measured using a standard grading scale. In an embodiment, the standard grading scale is:

| **Grade** | **Description of Grade** |
|---|---|
| 0 | No adhesion |
| 1 | Avascular adhesions |
| 2 | Vascular adhesions separable by blunt dissection |
| 3 | Vascular adhesions separable by sharp dissection |
| 4 | Vascular adhesions contiguous with the underlying tissues and organs, inseparable without damaging the underlying tissues and organs. |

In some embodiments, the administering is performed by spraying. In an embodiment, the spraying can be on the entire area of a peritoneum or a portion of the area of the peritoneum. In some embodiments, the administering is prophylactic.

In some embodiments, the method includes applying the composition according to some embodiments of the present invention on the surgical field, wherein the composition is liquid. The composition may be sprayed on (direct application) the entire surgical field or targeted as per the discretion of one skilled in the art (e.g. a surgeon). This can be done by any standard method (e.g., but not limited to, using a spray bottle, a pressurized gas spray system such as is used for fibrin sealants, a spray tip adjustment attached to a syringe, etc.) If performed in the manner described herein, the application of this extract will facilitate rapid clotting of active sites where minor bleeding may still be prevalent, thus reducing further bleeding, and bringing about a thin but dense fibrin covering. Non-bleeding, pristine, naive and/or undamaged tissue would not interact with the clotting factor extract in the absence of activating proteins such as TF. The end result of this medical use is a reduced potential for adhesion formation following abdominal or other surgery, e.g., a reduced amount of adhesions, a reduced adhesion severity, a reduced adhesion area, or any combination thereof.

The composition comprises a therapeutically effective amount of the composition according to some embodiments of the present invention, and optionally a pharmaceutically acceptable excipient.

In some embodiments, the surgery comprises abdominal surgery. The surgery may be laparoscopic surgery. In some embodiments, surgeries include spinal surgery, pelvic surgery, tendon surgery, cardiac surgery, appendage surgery including carpal tunnel surgery and the like. In the case of abdominal surgery, for example, the composition may be administered by spray to the abdominal cavity to prevent or reduce the formation of peritoneal tissue adhesions.

Without being bound to theory, the composition according to some embodiments of the present invention, when in contact with blood, or extravasated fibrinogen, can form a thin and dense fibrin layer, but only on the leaky vessels or injured tissue, which is able to function as a hemostatic layer and barrier to formation of adhesions. The formation of the fibrin layer may be attributed to the presence of tissue factor (TF). TF is expressed by cells which are normally not exposed to flowing blood such as sub-endothelial cells (e.g. smooth muscle cells) and cells surrounding blood vessels (e.g. fibroblasts). Injury to the blood vessel, e.g. during surgery, exposes this protein to interact with the composition and with the endogenous fibrinogen. The fibrin layer formed in this manner would be susceptible to plasminolysis, the natural process of fibrin removal from healing tissue. Without being bound to theory, any excess composition contacting an intact organ, tissue, or vessel, would be innocuous as the composition according to some embodiments of the present invention is limited in its ability to clot in the absence of certain clotting factors, for example Tissue Factor which greatly facilitates and accelerates blood clotting. Absence of excess fibrin-based clot at the surgery or wound site greatly reduces the risk of adhesion formation.

In some embodiments of the method, composition or use the subject is a mammal, e.g., a human. Veterinary methods are further encompassed by the present invention.

### A Kit (the kits described below in this section of the disclosure are not covered by the claimed invention)

The present disclosure provides a kit to produce a composition according to some embodiments of the present invention, comprising:
(a) a anion exchange column,
(b) a plurality of syringes, wherein each syringe of the plurality of syringes is configured to hold a wash solution, an elution solution, or an eluate,
(c) an eluate collection container,
(d) the wash solution,
(e) the elution solution, and
(f) instructions for preparing the composition according to some embodiments of the present invention,.
   wherein (a) to (e) are preassembled.

In some embodiments, the kit according to some embodiments of the present disclosure further comprises:
(g) a blood collection container, and
(h) a plasma collection apparatus.

In some embodiments, (a) is sterile.

In some embodiments, the composition comprises proaccelerin (factor V), and at least one factor selected from the group consisting of: prothrombin (factor II), proconvertin (factor VII), and Stuart-Prower factor (factor X),
wherein an amount of the proaccelerin in the composition is between 75% to 750% compared to an amount of proaccelerin in a blood plasma, and
wherein an amount of the at least one factor is from 150% to 3000% compared to an amount of the at least one factor in the blood plasma;
wherein the amount of proaccelerin and the at least one factor in the composition is determined by extrapolating an observed activity of the composition at a concentration of 500mM NaCl, using a prothrombin complementation assay using a standard curve constructed using the blood plasma.

In some embodiments, the composition further comprises fibrin stabilizing factor (factor XIII), wherein the concentration of the fibrin stabilizing factor in the composition is between 0.01 IU/mL to 100 IU/mL.

In some embodiments, the kit further includes components for both blood collection and plasma preparation. Therefore, provided herein is a kit for the preparation of the composition according to some embodiments of the present invention from an autologous or single donor comprising
a. a blood collection vessel;
b. plasma collection apparatus;
c. wash buffer and wash buffer article;
d. elution buffer and elution buffer article;
e. anion exchange column or manifold;
f. eluate collection container optionally containing dilution buffer; and
g. instructions for manufacturing the composition according to some embodiments of the present invention.

In some embodiments, the kit is sterile. In some embodiments, the anion exchange column is sterile.

In some embodiments, a wash buffer article is a device that is used to apply wash buffer to the column and an elution buffer article is a device that is used to apply elution buffer to the column. In some embodiments, the wash buffer device and the elution buffer device are independent syringes.

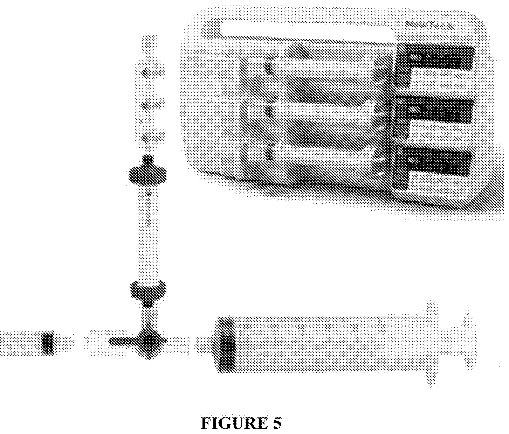

In some embodiments, the kit of the present disclosure is shown in Figure 5.

In some embodiments, by utilizing a small, hand-held pre-packed chromatography ion exchange column (e.g., but not limited to, HiTrap columns by GE) or filter (such as, but not limited to, a NatriFlo^{®} HD-Q Membrane Adsorbers by Natrix Separations Inc.) or a ready to pack mini-column combined with sterile pre-equilibrated resin that may be combined to rapidly form such a column, an amount of concentrate from plasma (i.e. clotting extract) sufficient for a single surgery, is prepared. Simple buffers and polymers, plastic or glass syringes and vials are used, and no cumbersome devices or biological or pharmaceutical agents are required. No lab machinery, except for a centrifuge in some of the embodiments, is required. A simple syringe pump and a valve may be incorporated to facilitate automatic or semi-automatic preparation. The kit may be delivered sterile and is engineered so that the entire process maintains the required sterility for the operating theater. No terminal sterilization is required. When utilizing autologous plasma or single-donor plasma without solvent-detergent treatment, the lipid components of the plasma such as phospholipids, platelets or platelet fragments, and microvesicles, are not destroyed in the process. The labile factor V (factor V) is retained at sufficient concentrations due to the rapid processing, and immediate titration of salt concentration immediately upon production of the clotting factor complex. The entire process is rapid, performed within up to 1-2 hours, and can be performed at the bedside, in the operating room, or at an adjacent facility (e.g. the hospital's blood bank) prior to or during the surgery.

In some embodiments, the chromatography ion exchange column is an anion exchange column. The anion exchange column can be strong (e.g., but not limited to the Q anion exchanger moiety) or a weak (e.g., but not limited to, diethyl-aminoethyl ("DEAE") moiety). The crosslinker to the Q or DEAE moiety can comprise any commercially available crosslinker. Additionally, the resin base (e.g., but not limited to, acrylic, agarose, dextran, etc.) can be any commercially available resin base.

In some embodiments, the chromatography ion exchange column is sterilized. In some embodiments, the chromatography ion exchange column is sterilized by using the method disclosed in WO2015109246. In some embodiments, the chromatography ion exchange column is sterilized by using the method disclosed in J.S. Moore et al. (1996), "Protection of Protein A-Sepharose Columns Irradiated to Sterilization Doses" Radiat. Phys. Chem. (47):1, pages 161-165.

In some embodiments, a plasma bag (e.g., a flexible container) is connected to a system containing a syringe pump for withdrawing the plasma from the bag via tubing, such as, but not limited to, a standard infusion set, needle, or other acceptable type of tubing.

The separation media (e.g., resin or gel) inside the column is functionalized with chemical groups designed to bind to and retain anionic proteins and compounds. Typical groups are "Q", "QAE", and "DEAE". One skilled in the art would appreciate that all of these are anion exchangers. Typical such columns are produced commercially by, e.g., but not limited to, GE, Natrix Separations Inc., TOSOH-HAAS, and other companies, or may be custom produced. Ready-to-pack columns, e.g. empty columns in combination with sterile pre-equilibrated resin that may be rapidly combined, may also be used. The nature and type of the functionalized media may vary between manufacturers and may include, but is not limited to, Sephadex^{™}, agarose, polyacrylamide-based gels, methacrylate-based resins, and other polymers and configurations.

In some embodiments, the syringe is then attached to an anion exchange manifold such as a column, containing separation media functionalized with positively charged groups. The amount of resin may be dictated by the volume of plasma, for example about 1:1 to 50:1 plasma:resin.

In some embodiments, the syringe is pre-attached to the a pre-attached kit, where the kit comprises: (a) a sterile anion exchange column, (b) a plurality of sterile syringes, where each sterile syringe of the plurality of sterile syringes is configured to hold a sterile wash solution, a sterile elution solution, or an eluate, (c) a sterile eluate collection container, (d) the sterile wash solution, and (e) the sterile elution solution.

While higher scale chromatography equipment (e.g. columns) are designed to be driven using pumps and separation machinery (e.g. FPLC), the small scale pre-packed columns, filters, or other similar manifolds, can be driven using the force exerted by a human hand. In some embodiments, the method of making the composition of the present invention is performed manually. In some embodiments, the method of making the composition of the present invention is automated. In some embodiments, the automated method can provide higher yields than the manual method, e.g., but not limited to 5%, 10%, 15%, 20%, 25%, 30%, 35%, 40%, 45%, or 50% higher yields of e.g., but not limited to, proaccelerin (factor V), prothrombin (factor II), proconvertin (factor VII), or any combination thereof. In some embodiments, the automated method can further provide higher yields than the manual method, e.g., but not limited to 5%, 10%, 15%, 20%, 25%, 30%, 35%, 40%, 45%, or 50% higher yields of e.g., but not limited to, Stuart-prower factor (factor X), fibrin stabilizing factor (factor XIII), or any combination thereof.

In some embodiments, a mechanical aide, for example, but not limited to, a specialized syringe and/ or pump can be used.

In some embodiments, the AEX manifold is attached via a one way valve, which allows the syringe pump to pull plasma from a flexible container such as a plasma bag, and then push it through another one-way valve by purging the syringe into the AEX manifold or column.

In some embodiments, the AEX manifold is attached via a 3-way stopcock in a way that allows the withdrawal of solutions from syringes into a working syringe placed in a syringe pump and then the driving of the contents through the anion exchange manifold using the power of the syringe pump.

In some embodiments, the three inlets of the 3-way stopcock of the valve are connected to 1) a working syringe in a syringe pump, 2) syringes that contain the plasma, the wash solutions, elution solutions and any other solutions required, and that are removed after the introduction of these solutions into the working syringe, 3) the anion exchange manifold. The valve is manipulated to allow flow from the interchanging solution-containing syringes into the working syringe by the pulling of the plunger of the working syringe by power of the syringe pump. Once a sufficient amount of plasma or solution has been loaded into the working syringe the valve is manipulated again to allow the flow from the working syringe into the anion exchange manifold by the pushing of the working syringe plunger by power of the syringe pump. Another syringe, waste bag, or container may be attached to the exit of the anion exchange manifold in order to collect wastes (e.g. plasma that has gone through the anion exchange manifold and wash solutions), or to collect the eluate. Such a system allows an almost hands-free, automatic or semi-automatic performance of the kit and method. Another advantage of such a system is that it requires little or no manual manipulation of the anion exchange manifold except for the attachment or detachment of syringes that can be performed holding the body of the syringe and without any physical contact with the inlets and outlets. A pre-assembled kit would require almost or virtually no manual manipulation of the syringes or AEX manifold or column, thereby reducing breakage of the components of the kit and/or reducing contamination, and/or reducing labor required by or personnel that are typically highly occupied in performing a large number of procedures during the operation.

The removal of larger cells while maintaining the smaller platelets, fragments, and microvesicles, may be achieved by use of filters. In one embodiment, a filter of about 1-20 um (e.g., 5-10 um) on, e.g., but not limited to, the 3-way stopcock between the stopcock and the syringe used to aspirate the plasma. Various filters have been devised to be used with syringes or luer attachments and are available commercially (e.g. blood aspiration needles with an embedded 5 um filter; luer-attachment filters of fixed pore size that are commercially available from different materials such as PVDF, cellulose acetate, PES, and other materials; and other filtration solutions). Such a filter would remove the larger cellular components (RBCs, WBCs) while allowing the entry of the smaller components (e.g. platelets, microvesicles). Another possibility is the use of such a filter as an entry filter in the column. A second filter may be attached to retain these particles within the column. The second filter may be attached at the outlet of the column and may have a pore size of typically below or equal to 1um. Commercial filters exist in different sizes that fit this category, e.g., but not limited to, 1 um, 0.8 um, 0.65 um, 0.45 um, 0.22 um, and 0.1 um. The filter may also be the outlet filter from the column. It would also be appreciated by one skilled in the art, that using typical size resin (e.g., but not limited to, about 10-200 um, usually about 40-100 um), even a packed (compressed) resin bed within the column would leave sufficient space for micron-size particles such as platelets or microparticles to intercalate in the column, and therefore elute with the plasma. In order to obtain the particulate components (e.g. platelets and microvesicles), the column arrayed in this way, would be eluted from the direction of the smaller pore-size filter (i.e., the 2^{nd} filter), in the reverse direction as the loading and washing direction, thus driving the eluate back through the larger pore-size filter (i.e., the 1^{st} filter). Alternatively, the filters may be dismantled for elution.

In some embodiments, it is also possible to obtain, using the method herein, a substantially particle-free extract that may be advantageous in some circumstances, by eluting in the same direction as the loading and washing direction, thereby forcing the eluate through the small pore-size filter (i.e. the 2^{nd} filter). It is also possible to achieve this in many other ways such as by utilizing a small pore size filter (<=1um,) to aspire the plasma, or loading the column with the filters arranged in the reverse order (i.e., the small pore filter, <=1um, as the entry filter), or both, or simply loading the column from the opposite side, or by utilizing a small pore-size filter for an additional final filtration step prior to applying the material.

In some embodiments, the elution vessel is a spray bottle. In some embodiments, the elution vessel is a syringe and spraying is achieved by means of a spraying attachment. In some embodiments, an attachment e.g., spray head, with or without the option to influx pressurized gas to release an aerosolic vapor, is attached to the syringe in order to maximize the delivery surface of the mixture. The elution syringe may therefore be attached to the exit of the column in the orientation required to directly collect the mixture for dispensing.

In some embodiments, the elution vessel contains (*a priori* or is added) a stabilizer of the group of coagulation factor inhibitors such as heparin (less than 0.1 IU/ml), arginine or lysine (e.g., about 1-100 mM, about 5-80 mM, about 10-40mM) (Stief, TW, Clin Appl Thromb Hemost 2007.13:2, 146-153), or any other inhibitors (e.g. benzamidine). Without wishing to be bound to theory, small amounts of inhibitor are sufficient to prevent activation of the clotting complex prematurely by inhibiting low level activation or activity of these zymogens, but are insufficient to prevent concerted activation of the clotting factors (e.g. by calcium addition or contact with tissue factor). In addition, small amounts of these inhibitors will be diluted in the blood to marginal levels. Direct thrombin inhibitors such as Dabigatran or factor Xa inhibitors such as Edoxaban, which are found in clinical use, may also be used.

In some embodiments, the elution solution is typically a similar solution to the washing solution, except that the concentration of the ions (e.g. NaCl ions) is significantly higher. Typical elution solution "strengths" are about 150-2000 mM NaCl, typically about 200-1000 mM NaCl, more typically about 250-800 mM NaCl. The required elution strengths depend on the anion exchange moiety and carrier particle type.

In some embodiments, the volume of the elution solution may be about 0.5-3 column volumes, typically about 0.75-2.5 column volumes, e.g., about 1-2 column volumes.

In some embodiments, the package or packages in which the kit components are provided prior to use, may include sterile components and/or hermetically sealed containers. The package is labeled, and the label may bear a notice as prescribed by a governmental agency, for example a national Food and Drug Administration (FDA), which notice is reflective of approval by the agency under Federal law, of the manufacture, use, or sale of the package for human use. Regulations vary from country to country, but individual procedures are well known to those skilled in the art and the compositions and methods provided herein comply accordingly.

In some embodiments, the composition is an aqueous composition for topical administration, e.g., packaged for use as a spray. The container may be a spray bottle with, for example, a broad nozzle to spray a large area or alternatively a cannula for localized spraying. The size of the nozzle, cannula and/or spray droplets may be adjusted depending on the application.

Such a kit is useful for the preparation of the composition according to some embodiments of the present invention when the plasma is provided, for example, from a commercially available source. Although, separate wash buffer article and elution buffer articles may be present in all the disclosed kits, it is possible that a single article will be provided in one or more of the kits and said article will serve as wash buffer and elution buffer article.

In some embodiments, the kit is adapted for the preparation of the composition according to some embodiments of the present invention from a donor, comprising:
a) a blood collection vessel (e.g. tube);
b) plasma collection apparatus;
c) wash buffer and wash buffer article
d) elution buffer and elution buffer article;
e) anion exchange column or manifold;
f) eluate collection container, optionally containing dilution buffer; and
g) instructions for preparing the composition according to some embodiments of the present invention.

In some embodiments of the kits of the present disclosure, the wash buffer and elution buffer articles may independently be a syringe or a vessel able to be interconnected with the system.

In some embodiments, the kit may be packaged in a single package or may be provided in multiple parts.

The present invention is further illustrated, but not limited by, the following examples.

### EXAMPLES

### EXAMPLE 1: PREPARATION OF A COMPOSITION INCLUDING AT LEAST ONE CLOTTING FACTOR

### Overview:

The purification and concentration of the composition according to some embodiments of the present invention was prepared by first bleeding a mammal (e.g., but not limited to, a cow, pig, horse, etc.) to collect between 1000-2000 ml blood. The plasma was prepared by centrifuging the blood for 10 to 15 minutes at 3000 RPM (roughly 1700 RCF). The plasma was collected, filtered, and passed through an AEX column or other manifold to concentrate the anionic blood factors. Functional testing was performed for remaining levels of factors II, VII, X, and the associated co-factor pro-accelerin (V).

In another example, human source plasma is used to prepare the composition comprising at least factor V.

In another example, a fibrinogen-depleted ESC is prepared by altering the wash and elution buffer and/or including one or more biocompatible polymers in the centrifugation step.

Specific examples are provided below.

### Experiment 1: Fibrinogen depletion using PEG in the centrifugation step.

Two pigs were bled and blood was collected in ACD-A anticoagulant solution (1 liter per pig).

Blood was centrifuged in 50 ml tubes to separate the cellular and plasma phases, and the plasma collected. Plasma was stored frozen until use (approximately one month at -20 deg C). Plasma was then thawed, and centrifuged again under the same conditions with different concentrations of different average molecular weight (MW) PEG polymer solutions. A Clauss assay was performed to analyze the remaining levels of fibrinogen in plasma compared to a standard curve generated with plasma centrifuged without PEG (see, e.g., Clauss method ("thrombin clotting time method") described in Morse, E.E. and Viswanathan, U. "Determination of Fibrinogen in Plasma," Annals of Clinical and Laboratory Science, 1978, 8: 438-441, and the reference: Clauss A, "Rapid Physiological Coagulation Method for the Determination of Fibrinogen, "Acta Haematol, 1957, 17:237-46.).

The results obtained are shown in Table 1. Low MW PEG (e.g., 5% PEG 1450), or high MW PEG at low concentrations (e.g., 1% PEG 6000), were inefficient at removing fibrinogen. By manipulating the MW and concentration of PEG, however, both partial and essentially complete fibrinogen removal (undetectable fibrinogen levels using the Clauss assay) was achieved (e.g., 5% PEG 3350, 2.5% PEG 6000, and 5% PEG 6000).

**Table 1: Average relative levels of plasma Fibrinogen after PEG precipitation.**

| **PEG treatment** | **Remaining Fibrinogen** |
|---|---|
| 5% PEG 1450 | 109%^{∗} |
| 5% PEG 3350 | 41% |
| 1% PEG 6000 | 106%^{∗} |
| 2.5% PEG 6000 | 63% |
| 5% PEG 6000 | 0% |

| | |
|---|---|
| ^{∗} Extrapolated Fibrinogen levels of higher than 100% of control plasma are due to the limited accuracy level of the assay. | |

### Experiment 2: Preparation of a composition comprising at least one clotting factor from porcine plasma.

A HiTrap Q FF 1 ml column was prepared, and porcine plasma (15-20 ml) was passed manually through the column (using an attached syringe). The column was then washed with a low salt citrate buffer, and then eluted in a high salt buffer (500-750 mM). Three different elution conditions were tested and factors II, V, VII, and X levels were tested using a modified PT (prothrombin) assay with the respective factor-depleted plasma. A final amount of 4 ml extract was prepared after dilution with citrate buffer.

Table 2 lists the relative yields of factor II (FII), factor V (FV), factor VII (FVII), and factor X (FX) of three independent experiments performed after manual preparation of a composition comprising at least one clotting factor from pig plasma using a pre-packed column.

**Table 2:**

| **Elution strength** | **FII** | **FV** | **FVII** | **FX** |
|---|---|---|---|---|
| 500mM | 71% | 9% | Not tested | Not tested |
| 600mM | 116%^{∗} | 11% | 106%^{∗} | 80% |
| 750mM | 105%^{∗} | 14% | 102%^{∗} | 84% |

Table 3 lists the functional concentration (i.e., clotting factor activity) of the derived composition in the experiments detailed above, which were manually prepared from pig-plasma using a pre-packed column. Higher yields were obtained with higher elution strengths. Using 20 ml of input plasma, clotting factors could be concentrated to between 4-5 times their endogenous levels found in plasma. Regarding labile factors: factor V recovery was greater than 9% in all runs, and factor VII was recovered completely.

**Table 3:**

| **Elution strength** | **Factor II** | **Factor V** | **Factor VII** | **Factor X** |
|---|---|---|---|---|
| 500mM | 267% | 35% | Not tested | Not tested |
| 600mM | 578% | 53% | 531% | 400% |
| 750mM | 523% | 71% | 509% | 420% |

| | | | | |
|---|---|---|---|---|
| ^{∗} Extrapolated factor levels of higher than 100% of input plasma are due to the limited accuracy level of the assay. | | | | |

### Experiment 3: Preparation of a composition comprising at least one clotting factor from human plasma.

Human source plasma was used with the same column, and loaded as described in examples above. Various parameters (wash, elution, load amounts) were varied between experiments. Tables 4, 5, and 6, list the different conditions tested and results thereof.

Table 4 details the testing parameters (loading volume, wash strength, elution strength, final volume, optional addition of 2mM MgCl₂ or solvent-detergent (SD) treatment) used in the experiments (run 4, run 5, run 6, run 7, run 8, and run 9).

**Table 4:**

| | **Run 4** | **Run 5** | **Run 6** | **Run 7** | **Run 8** | **Run 9** |
|---|---|---|---|---|---|---|
| **Loading volume** | 20ml | 20ml | 20ml | 20ml | 25ml | 25ml |
| **Wash strength** | 100mM | 100mM | 75mM | 50mM | 50mM | 0 mM^{∗} |
| **Elution strength** | 750mM | 750mM | 600mM | 750mM | 750mM | 750mM |
| **Final volume** | 4ml | 4ml | 6ml | 6ml | 6ml | 6ml |
| **Other treatments** | | 2mM MgC12 | | | | SD treatment |

| | | | | | | |
|---|---|---|---|---|---|---|
| ^{∗} 10 column volumes (10 ml) of buffer without salt addition was used to wash the column after SD treated plasma was loaded. | | | | | | |

Table 5 details the clotting factor recoveries from compositions comprising at least one clotting factor obtained manually using a pre-packed column.

**Table 5:**

| | **Concentration vs. input plasma** | | | | | |
|---|---|---|---|---|---|---|
| | Run 4 | Run 5 | Run 6 | Run 7 | Run 8 | Run 9 |
| FII | 480% | 472% | 452% | 330% | 427% | 422% |
| FV | 40% | 16% | 32% | 54% | 40% | 15% |
| FVII | 482% | 449% | 239% | 270% | 281% | 374% |
| FX | 552% | 519% | 446% | 332% | 355% | 408% |

| | **Clotting factor Recovery** | | | | | |
|---|---|---|---|---|---|---|
| FII | 96% | 94% | 136% | 99% | 102% | 101% |
| FV | 8% | 3% | 10% | 16% | 10% | 4% |
| FVII | 96% | 90% | 72% | 81% | 67% | 90% |
| FX | 110% | 104% | 134% | 100% | 85% | 98% |

Table 6 details the fibrinogen recovery from human compositions comprising at least one clotting factor obtained manually using a pre-packed column.

**Table 6:**

| | **Run 4** | **Run 6** | **Run 7** | **Run 9** |
|---|---|---|---|---|
| **Relative to input plasma** | 7.2% | 7.3% | 6.0% | 38% |
| **Fibrinogen recovery** | 1.4% | 1.5% | 1.8% | 8% |

Factor V levels after SD-treatment (performed by 1% TX-100 and 1% NP-40 addition) were at or above 4% recovery or 15% functional factor V levels in the extract compared to the input plasma levels. Other factors were recovered with a higher than 90% efficiency, indicating that SD-treated plasma can be utilized in this method and kit.

Recovery of enzymatic factors was at or above 67%, and, in some experiments, above 80%, including the labile factor VII, except for when elution strength was reduced.

The concentration factor for the PPSB clotting factors was selected to be between approximately 3 to 5-fold compared with the levels found in untreated plasma, though this is subject to change by modifying the final volume and/or the load volume of the input plasma. For example, if the elution volume is reduced by half the clotting factors would be between 6 and 10 fold compared with the levels found in untreated plasma.

The removal of Fibrinogen was higher than 98% when performing a salt-wash step, but only approximately 92% when washing with salt-free buffer. Thus, it is possible to remove most of the Fibrinogen simply by performing a low-salt wash step, or conversely, retain significant concentrations thereof. Low salt wash buffers can include, but are not limited to, 50 mM to 150 mM NaCl.

This data were acquired using a pre-packed column with a syringe, driven manually using the power of a human hand.

### EXAMPLE 2: IN VIVO TESTING OF COMPOSITIONS COMPRISING AT LEAST ONE CLOTTING FACTOR

### Overview:

The *in vivo* experiments were performed in rats. The small size of rats, however, precludes the withdrawal of sufficient amounts of blood for manipulation with the proposed kit (requires taking >20% of blood volume). Accordingly, human plasma was processed to use in 5-10 rats (approximately 1 ml per rat, equivalent to approximately 50-100 ml of initial blood for all rats).

The experiment was performed by anaesthetizing rats and expositing the rats' peritoneum. An adhesion forming manipulation was the performed on the rats (cecal abrasion). After the cecal abrasion was performed, the composition according to some embodiments of the present invention was applied to the site of the abrasion and around it. Control rats were treated with saline. Two weeks afterwards, animals were sacrificed, and graded blindly for adhesion scores. Representative images are taken for comparison.

### Animal experiments:

Six (6) adult white Wistar rats were anaesthetized and a midline incision was made, after which the cecum was taken out by holding with a wet gauze using gloves, and gently abraded 15 times with wet gauze, in order to stimulate the formation of mild adhesions (grades 1-2 according to AFS standard scoring). Four control animals were sutured, and two test animals were treated with 1 ml of test composition generated from human plasma at approximately 5X concentration of clotting factors II, VII, and X, and 15% factor V (compared to input plasma). Over 95% of the Fibrinogen was depleted from the input plasma. The composition was applied as a spray using a 1 ml syringe with a 33G needle.

Test animals were then sutured in the same way as the controls. Two weeks later, animals were terminally anaesthetized and opened along the incisional scar and graded for adhesions. Three (3) of four (4) control animals showed grade 1-2 adhesions. None of the test animals showed any sign of adhesion formation. The composition comprising at least one clotting factor thus shows a capacity for the prevention of surgical adhesion formation. Table 7 lists the results of this cecal abrasion rat model experiment.

**Table 7:**

| **Animal number** | **Treatment/Control** | **Adhesion grade** | **Adhesion length** | **Location** |
|---|---|---|---|---|
| 1 | Control | 1-2 | 0.5 cm | Incisional scar |
| 2 | Control | 1 | Point adhesion | Omentum-small pelvis fat pad |
| 3 | Treatment | NONE | - | - |
| 4 | Treatment | NONE | - | - |
| 5 | Control | NONE | - | - |
| 6 | Control | 2 | 1.5 cm | Incisional scar |

| | | | | |
|---|---|---|---|---|
| Representative images are shown in Figures 2A-2C. | | | | |

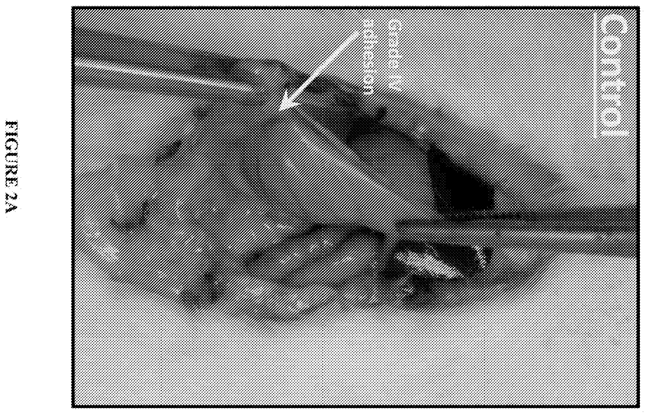

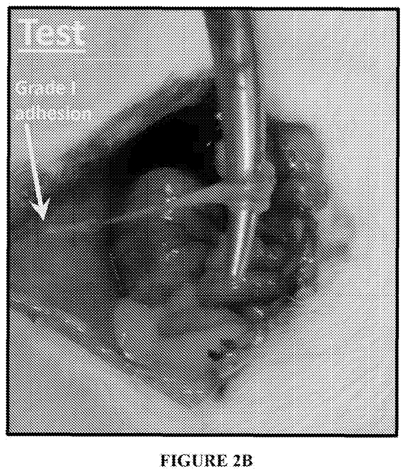

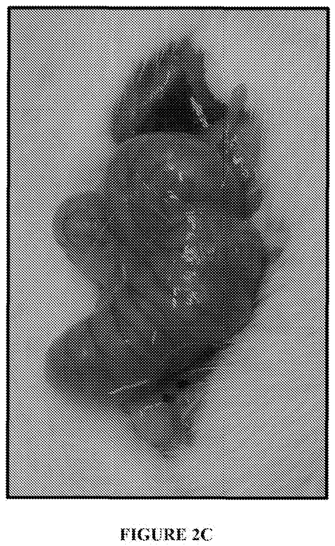

Adhesions (where present) are held towards the camera with forceps: Figure 2A Animal 1 (control) displaying a grade IV adhesion [cecum to peritoneum]; Figure 2B displaying a grade I adhesion in an animal treated with fresh plasma-derived concentrate. The adhesion is between fat tissue and the peritoneal wall, but the cecum is free of adhesion; Figure 2C shows the same animal as in figure 2B (test). In order to better illustrate the lack of any adhesions on the cecum, it was pulled out of the abdominal cavities.

The results show that the ESC is effective at reducing adhesion grade and number.

### EXAMPLE 3: EVALUATION OF ANTI-ADHESION TECHNOLOGY

The following experiments were performed to assess the prevention of forming surgical adhesions following an induction model of cecal abrasion with peritoneal sidewall defect.

### STUDY END POINTS:

The experiment was terminated 13-17 days after an adhesion induction protocol and treatment with different variants of the treatment article including both fresh and freeze/thawed material, and material with addition of an activator (CaCh), and a positive control Fibrin Sealant group. Animals were sacrificed and adhesions were graded for severity according to a standard American Fertility Society (AFS) grading scheme.

### RATIONALE FOR EXPERIMENT DESIGN / MATERIALS AND PREPARATION METHODS

### Principle of the test

Adhesion formation was induced by cecal abrasion and the generation of a peritoneal sidewall defect of 2 x 1 cm in juxtaposition to the abraded cecum. The tested material was applied both to the cecum after abrasion, and to the peritoneal sidewall defect. Adhesions were graded by their appearance and tenacity post-mortem according to the standard AFS scoring scale.

### Initial considerations

All available information on the test item was considered, such as previous efficacy data of similar materials (e.g. Fibrin sealants). The test item is a blood-derived concentrated extract with no other chemically active components and is therefore unlikely to cause any pain and distress, or have any corrosive or irritant effects.

### Justification for the selection of test system

The selected rodent species is the rat. Healthy young adult animals of commonly used laboratory strains were employed. This rat model is an initial step providing early information about the efficacy of the subject composition for adhesion prevention.

### Rationale for test group size

Two experiments were performed. A total of 16 rats in three groups were utilized in each experiment. Each experiment was comprised of a control group receiving only saline (5-6 animals), and two experimental groups. In experiment 1, these were either fresh or freeze/thawed material. In experiment 2, these were fresh material with CaCh activator (25mM CaCh, added 5-10 minutes prior to application) and Fibrin Sealant (FS; Evicel). Each test group included 5-6 animals. The total number of animals is based on previous studies demonstrating that this is the minimum number of animals per group that produces significant information. Adhesion severity is variable between individual animals.

### Justification for route of administration

Surgical adhesions form after significant trauma to the peritoneum such as was induced by opening of the abdominal cavity and abrasion of the cecum together with excision of the juxtaposed peritoneum (a 1x2 cm square). The test material was then applied onto the abrasion site and its immediate surroundings by dripping or spraying from a high-gauge (31) insulin syringe in a similar manner to that would be used in actual surgery.

### MATERIALS AND PREPARATION METHODS

### Experiment 1

Table 8 provides description of the treatments used in experiment 1.

**Table 8:**

| **Test No.** | **1** | **2** | **3** |
|---|---|---|---|
| **Test Item*** | A | B | C |
| **Batch No.** | Saline | Fresh extract - 5X concentrated | Frozen extract - 5X concentrated |
| **Physical State** | Solution | Solution | Solution |
| **Storage** | RT | RT | RT (after thawing) |
| **Manufacture Date** | Commercially produced valid batch | Up to 3 hours prior to experiment | Approximately 24 hours prior to experiment |
| **Expiry Date** | | N/A | N/A |

| | | | |
|---|---|---|---|
| ^{∗} Extracts are prepared from human blood plasma via AEX to desired concentration by reducing the volume | | | |

### Experiment 2

Table 9 provides description of the treatments used in experiment 2.

**Table 9:**

| **Test No.** | **1** | **2** | **3** |
|---|---|---|---|
| **Test Item*** | A | B | C |
| **Batch No.** | Saline | Fresh extract - 5X concentrated, with 25mM CaCl₂ activator added 5-10' prior to application | Evicel commercial Fibrin Sealant |
| **Physical State** | Solution | Solution | Solution |
| **Storage** | RT | RT | RT (after thawing) |
| **Manufacture Date** | Commercially produced valid batch | Up to 3 hours prior to experiment | 2011 |
| **Expiry Date** | | NA | ^{∗} Evicel Fibrin Sealant is highly stable at -20C storage conditions |

| | | | |
|---|---|---|---|
| ^{∗} Extracts are prepared from human blood plasma via AEX to desired concentration. | | | |

### EXPERIMENTAL MODEL - ANIMALS

Male Lewis rats were used in the following examples (obtained from Harlan Laboratories, Israel). The rats' body weight was 200-250gr at study initiation. The minimum and maximum weights of the group were within a range of ± 10 % of group mean weight. The acclimation period was 5 days. The rats were identified using permanent marker (up to 24hours experiment) and cage cards.

Animals handling was carried out according to the National Institute of Health (NIH) and the Association for Assessment and Accreditation of Laboratory Animal Care (AAALAC). Animals are housed in polysulfone (PSU) cages (4-6/cage), with stainless steel top grill having facilities for pellet food and drinking water in clear polycarbonate bottle; bedding: steam sterilized clean paddy husk was used and bedding material was changed along with the cage at least twice a week.

Animals were fed ad libitum a commercial rodent diet. Animals had free access to autoclaved drinking water obtained from the municipality supply. Animals were housed under standard laboratory conditions with adequate fresh air supply. Animals were kept in a climate controlled environment. Temperatures range was between 20 - 24°C and relative humidity (RH) is between 30-70% with 12 hours light and 12 hours dark cycle. Animals were inspected on arrival in order to fit the study. Following surgery, animals were inspected after 24 hours to ensure that they have recovered. Animals were maintained between 13-17 days post treatment in order to enable adhesion development and were then sacrificed. Routine inspection and animal husbandry was performed. This study was performed after approval by "The Israel Board for Animal Experiments" and in compliance with "The Israel Animal Welfare Act".

### TEST PROCEDURE

All tested animals were randomly assigned to respective treatment.

### EXPERIMENTAL DESIGN AND CONDITIONS: Route of Administration, dosage levels and volumes

### Constitution of Test Groups

Table 10 shows the constitution of test groups, number of animals, test material, volume and administration route used in the subject experiment. All solutions were used at room temperature.

**Table 10:**

| **Experiment no.** | **Group no.** | **Rat no.** | **Test article** | **Dose/Volume (ml/kg)** | **Route** |
|---|---|---|---|---|---|
| **1** | **1** | 6 | control | 1 ml | Applied on exposed peritoneum |
| **1** | **2** | 5 | FRESH | 1 ml | Applied on exposed peritoneum |
| **1** | **3** | 5 | FROZEN | 1 ml | Applied on exposed peritoneum |
| **2** | **1** | 5 | control | 1 ml | Applied on exposed peritoneum |
| **2** | **2** | 5 | FRESH+ CACL₂ | 1 ml | Applied on exposed peritoneum |
| **2** | **3** | 6 | FROZEN | 1 ml | Applied on exposed peritoneum |

The body weight of each rat was measured 3 days before the experiment or at the same day, prior to test administration.

Rats were treated with the test material by dripping or spraying from a high-gauge needle on the surgically induced adhesion site (the abraded cecum). Fibrin sealant was applied using two syringes to the same locale in simulation of actual application procedure of fibrin sealants.

The procedure was as follows: (1) 13-17 days following administration of test articles, animals were terminally anesthetized by pentobarbital. (2) The abdomen was opened and the peritoneum and cecum exposed. (3) Adhesions were graded according to a standard grading scale of 0 (no adhesions) to 4 (persistent adhesions that cannot be removed without damaging the underlying organs), according to standard AFS scoring. (4) Images of the exposed peritoneum, of the cecum, and of any adhesion, were recorded.

### RESULTS

No adverse effects were reported during this study. All animals in both control and test groups gained weight normally. No fever, apathetic behavior, lack of grooming, social isolation behavior, or deaths, were recorded. Table 11 displays the weight gain recorded for all animals in all the experimental groups (experiment 1 and experiment 2). All animals gained weight normally, and not a single animal lost weight. Average weight gain was 31.14 gr/week (+/- 0.36 gr). Specifically, for experiment 1, the pre-treatment measurement (body weight measured in grams) is dated 4/17/2016, while the post-treatment measurement (body weight measured in grams) is dated 4/24/2016 and 5/5/2016. For experiment 2, the pre-treatment measurement (body weight measured in grams) is dated 5/15/2016, while the post-treatment measurement (body weight measured in grams) is dated 5/21/2016 and 5/29/2016.

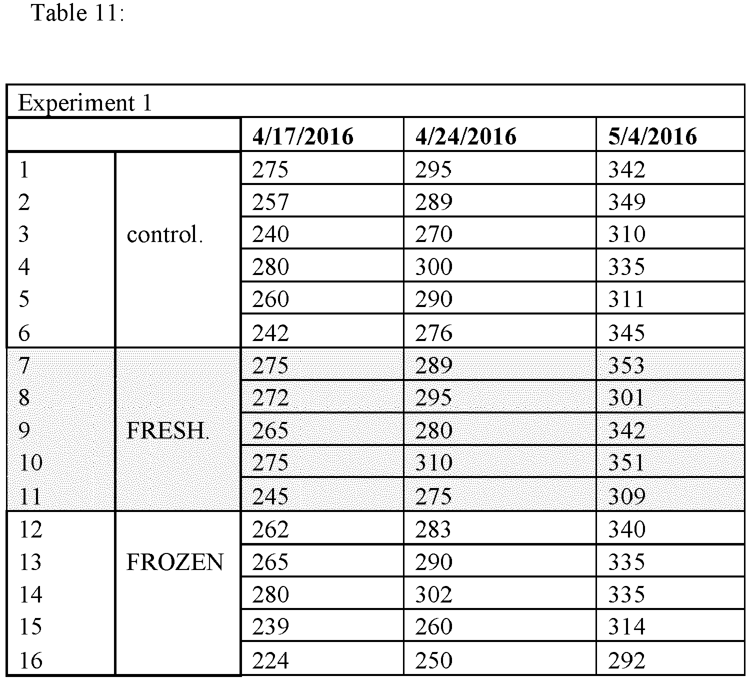

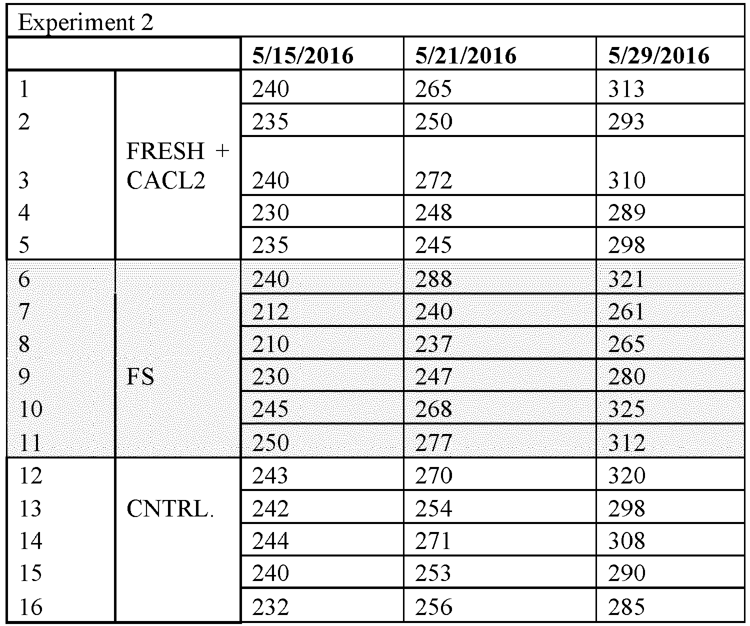

Table 11 shows the results of recording animal body weight during the experiments. All animals gained weight normally.

In the first experiment, the Control group (CNTRL.) received saline, treatment (FRESH) received fresh test material, and frozen treatment group (FROZEN) receiving the frozen test material. In the second experiment, the Control group (CNTRL.) receiving saline, the Calcium activator group (FRESH + CACL2) received fresh material with CaCh activator, and the Fibrin Sealant (FS) group, received Fibrin Sealant.

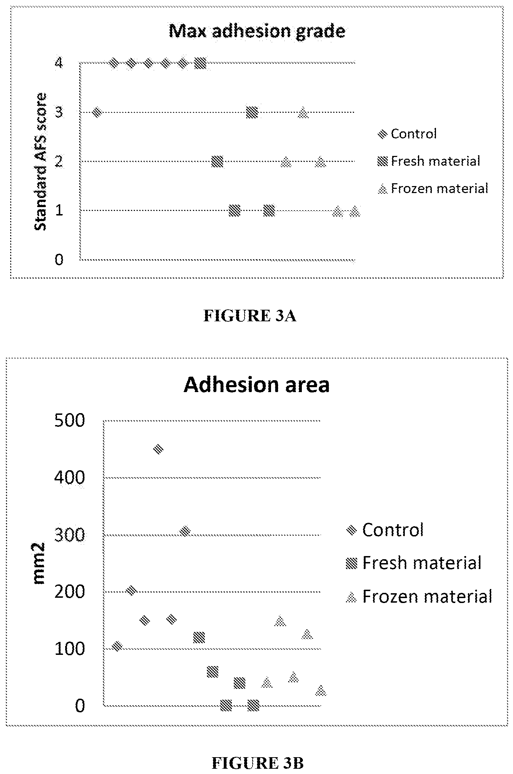

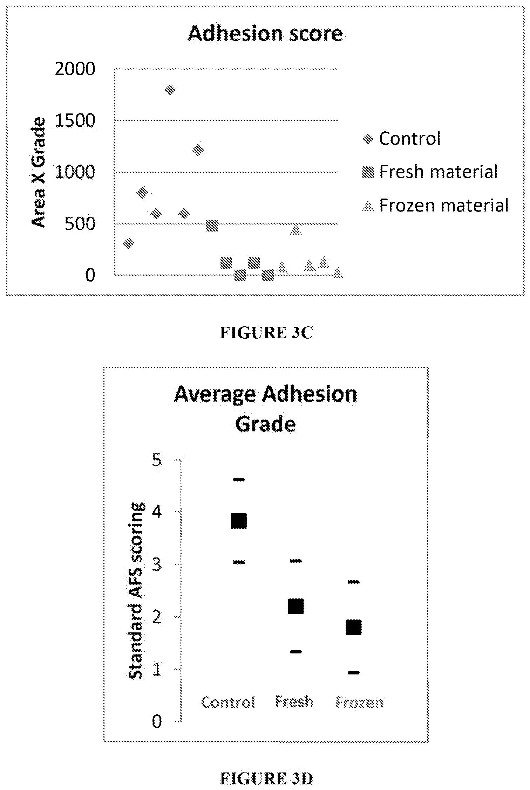

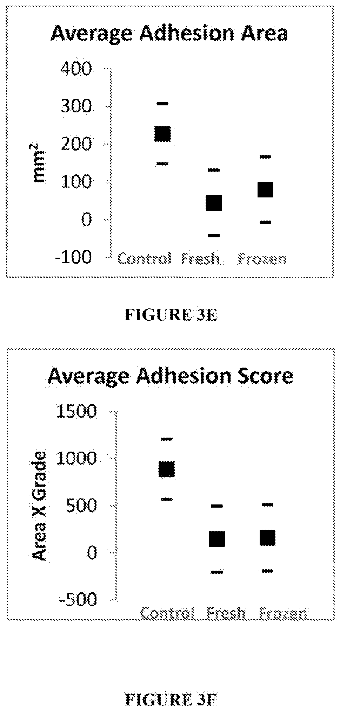

Figures 3A-F shows the results of the first experiment. Figures 3A-C show data plots for individual animals. Figures 3D-F show average data with 95% confidence intervals.

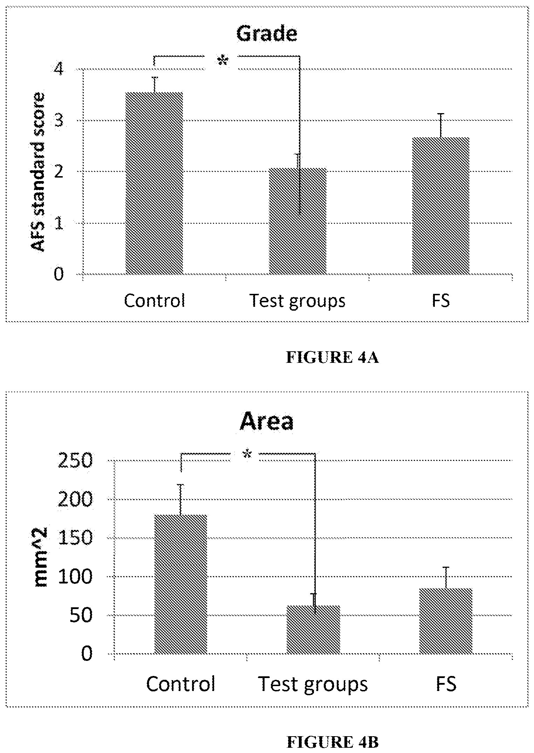

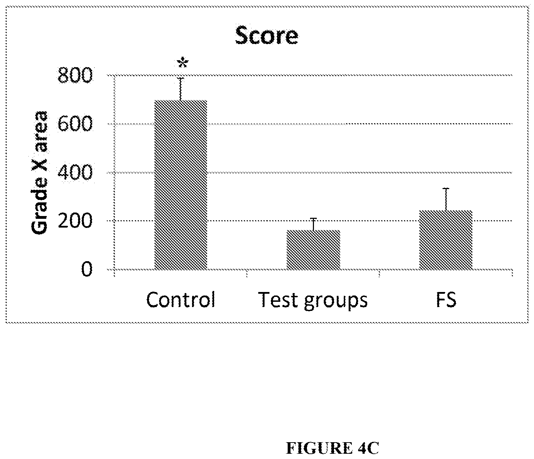

Figures 4A-C show average data for pooled test groups from both experiments 1 and 2 vs. fibrin sealant and control.

In the first experiment, control animals displayed large adhesions of the highest possible severity, while both fresh and frozen test groups were similarly efficient in reducing the severity (grade and area) of adhesions. The data from the first experiment showed a clear and statistically significant difference between the control and both test groups, with no significant differences between either test groups (Fig. 4A-C). Experiment 2 was designed to repeat the first experiment but add information on the biochemical pathways bringing about the reduction in adhesion severity. To this end the test material was prepared in the same manner as in experiment 1 (<10% differences in the activity levels of the different factors, not shown), but CaCh was added 5-10 minutes prior to application.

Calcium is an activator of the intrinsic pathway of coagulation. This pre-activation was performed in order to test our hypothesis as to the mode of action - the activation of the extrinsic pathway. The major difference between the pathways in terms of adhesion prevention is that the extrinsic pathway responds to tissue damage (via interaction with Tissue Factor), whereas the intrinsic pathway can be activated passively using calcium. The implication is that extrinsic pathway activation occurs only in the sites most prone to adhesion formation, whereas intrinsic pathway activation would be less specific regarding the site of action of the material. Improvement of the adhesion prevention activity of the test material with calcium addition would therefore indicate that the intrinsic pathway is involved in the biochemical pathway leading to adhesion prevention.

Another experimental group with a different biochemical mode of action was also tested, the application of Fibrin Sealant. Fibrin Sealants are plasma products with hemostasis activity. Fibrin sealants spread a thick and highly cross-linked fibrin layer where applied, thus potentially reducing permeability to fibroblasts that initiate the adhesion process.

The results showed similar results for the test material pre-activated with CaCh as the fresh and frozen test material tested in the first experiment (area and grade of adhesions for the CaCl₂ activated group were both between the values obtained using the fresh and frozen material tested in experiment 1, not shown). The three test groups, therefore, were pooled for further analysis (as were the control groups for both experiments), Figure 4A-C. The effects of the Fibrin Sealant (FS) group were less pronounced than the test material, and were not statistically significant for neither the severity (grade) nor the area of adhesions, compared to the control group, unlike the test material produced by the subject method and kit (although a trend towards reduction of adhesion severity was seen for the Fibrin Sealant group for all parameters tested).

Table 12 shows the individual data (adhesion area, grade of adhesion area, scale of grade and description) for all of the animals tested in experiment 1.

**Table 12:**

| **Animal number** | **Area (mm²)** | **Score (grade X area)** | **Grade** | **Description** |
|---|---|---|---|---|
| 1 | 105 | 310 | 3 | Control |
| 2 | 202 | 802 | 4 | Control |
| 3 | 150 | 600 | 4 | Control |
| 4 | 450 | 1800 | 4 | Control |
| 5 | 152 | 602 | 4 | Control |
| 6 | 307 | 1214 | 4 | Control |
| 7 | 120 | 480 | 4 | Fresh |
| 8 | 60 | 120 | 2 | Fresh |
| 9 | 1 | 1 | 1 | Fresh |
| 10 | 40 | 120 | 3 | Fresh |
| 11 | 1 | 1 | 1 | Fresh |
| 12 | 42 | 84 | 2 | Frozen |
| 13 | 150 | 450 | 3 | Frozen |
| 14 | 51 | 101 | 2 | Frozen |
| 15 | 127 | 127 | 1 | Frozen |
| 16 | 28 | 28 | 1 | Frozen |

Table 13 shows the individual data (adhesion area, grade of adhesion area, scale of grade and description) for all animals tested in experiment 2.

**Table 13:**

| **Animal number** | **Area (mm²)** | **Score (grade X area)** | **Grade** | **Description** |
|---|---|---|---|---|
| 1 | 53 | 156 | 3 | Fresh+CaCl2 |
| 2 | 103 | 303 | 3 | Fresh+CaCl2 |
| 3 | 2 | 2 | 1 | Fresh+CaCl2 |
| 4 | 163 | 476 | 3 | Fresh+CaCl2 |
| 5 | 0.04 | 0.04 | 1 | Fresh+CaCl2 |
| 6 | 8 | 8 | 1 | FS |
| 7 | 105 | 205 | 2 | FS |
| 8 | 73 | 193 | 3 | FS |
| 9 | 166 | 486 | 3 | FS |
| 10 | 26 | 78 | 3 | FS |
| 11 | 131 | 491 | 4 | FS |
| 12 | 152 | 602 | 4 | Control |
| 13 | 45 | 130 | 3 | Control |
| 14 | 200 | 800 | 4 | Control |
| 15 | 220 | 820 | 4 | Control |
| 16 | 0.1 | 0.1 | 1 | Control |

Inspection of the individual data for the rats analyzed in the experiments supported the conclusions of the analyses above (see, e.g., Tables 12 and 13). First, the adhesion induction protocol generated surgical adhesions: 8/11 (72%) control animals displayed the maximal adhesion grade (4), and 2/11 (18%) displayed grade 3 adhesions. Only one control animal displayed grade 1 adhesions (9%) in both experiments. Second, the test material, whether fresh, frozen, with, or without activation, prevented surgical adhesion formation. 60% of treated animals displayed only grade 1/2 adhesions, and only 1/15 (6.7%) displayed grade 4 adhesions. Third, Fibrin Sealant (FS) was less effective than the test material, as 2/6 (33%) of animals treated with FS displayed grade 1/2 adhesions, the rest displaying grades 3 and 4 adhesions. FS was also less effective in reducing the total area of adhesions after surgery.

### CONCLUSIONS

In summary, the material produced by the kit described herein reduces surgical adhesions.

The composition of the present invention reduces the amount of adhesions after frozen storage, and with or without pre-activation with CaCh. Furthermore, Fibrin Sealant was less effective than the proposed kit in adhesion prevention.

These results support that the composition of the present invention utilizes a novel biochemical mechanism to prevent the formation and reduce the severity of surgical adhesions.

### Biochemical activity of material used in this study

Table 14 shows the biochemical activity of the different factors in the ESC used in experiments 1 and 2. These experiments were performed using a 2.5 fold dilution of ESC.

**Table 14:**

| **Factor** | **FII** | **FV** | **FVII** | **FX** | **Fibrinogen** |
|---|---|---|---|---|---|
| Experiment 1 | 530% | 33%^{∗} | 488% | 527% | 20% |
| Experiment 2 | 562% | 39% | 448% | 496% | 20% |
| Relative (exp 2/exp 1) | 108% | 119% | 93% | 94% | 101% |
| ^{∗} factor V levels after the freeze/thaw cycle were reduced 55% (to 15% compared to input plasma) in experiment #1 | | | | | |

Absolute concentrations were approximately 5-fold for the enzymatic factors (e.g., factor II, factor VII, and factor X) and between 30-40% for factor V. The material was homogenous between experiments 1 and 2, with less than 10% difference for the enzymatic factor levels, and less than 20% difference for the cofactor V. Fibrinogen levels were depleted to approximately 20% of the input plasma levels in the concentrated material.

### EXAMPLE 4: MILD INDUCTION MODEL OF CECAL ABRASION

The following study was conducted to test the capacity of the treatment to prevent the formation of surgical adhesions following a mild induction model of cecal abrasion *without* peritoneal sidewall defect.

### STUDY END POINTS:

The study was terminated 15 days after an adhesion induction protocol and treatment with the treatment article (prepared fresh). Animals were sacrificed and adhesions were graded for severity.

### RATIONALE FOR EXPERIMENT DESIGN / MATERIALS AND METHODS

Relatively mild adhesion formation was induced by cecal abrasion but without the peritoneal sidewall defect. The tested material was applied both to the cecum after abrasion, and to the peritoneum on which the cecum was then overlayed prior to suturing of the animal. Adhesions were graded by their appearance and tenacity post-mortem.

The subject composition was previously used in a study to assess its effectiveness and safety in the cecal abrasion *with* peritoneal sidewall defect model. The model is less aggressive as it is identical to the previous model but without the sidewall defect generation. The purpose of this study was to assess the capability of the subject composition to prevent mild adhesions in a less aggressive model. This test was performed to evaluate a different potential clinical setting, of a mild surgical intervention.

A small group size was selected (6 animals total: 3 controls and 3 test animal) to minimize animal suffering.

Similar to the previous experiment, the test material was applied onto the abrasion site and its immediate surroundings by dripping or spraying from a high-gauge (31) insulin syringe to emulate the methods expected to be used in actual surgery.

### MATERIALS AND PREPARATION METHODS

Table 15 outlines the treatments used on rats in the following experiment.

**Table 15:**

| **Test No.** | **1** | **2** |
|---|---|---|
| **Test Item*** | A | B |
| **Batch No.** | Saline | Fresh extract - 5X concentrated |
| **Physical State** | Solution | Solution |
| **Storage** | RT | RT |
| **Manufacture Date** | Commercially produced valid batch | Up to 3 hours prior to experiment |
| **Expiry Date** | | N/A |

| | | |
|---|---|---|
| ^{∗} Extracts are prepared from human blood plasma via AEX to desired concentration by reducing the volume. | | |

Male Lewis rats were used in the following examples (obtained from Harlan Laboratories, Israel). The rats' body weight was 200-250gr at study initiation. The minimum and maximum weights of the group were within a range of ± 10 % of group mean weight. The acclimation period was 5 days. The rats were identified using permanent marker (up to 24hours experiment) and cage cards.

Animals handling was carried out according to the National Institute of Health (NIH) and the Association for Assessment and Accreditation of Laboratory Animal Care (AAALAC). Animals were housed in polysulfone (PSU) cages (4-6/cage), with stainless steel top grill having facilities for pellet food and drinking water in clear polycarbonate bottle; bedding: steam sterilized clean paddy husk was used and bedding material was changed along with the cage at least twice a week.

Animals were fed ad libitum a commercial rodent diet. Animals had free access to autoclaved drinking water obtained from the municipality supply.

Animals were housed under standard laboratory conditions with adequate fresh air supply. Animals were kept in a climate controlled environment. Temperatures range was between 20 - 24°C and RH is between 30-70% with 12 hours light and 12 hours dark cycle.

Animals were inspected on arrival in order to fit the study. Following surgery, animals were inspected after 24 hours to ensure that they have recovered. Animals were maintained between 13-17 days post treatment in order to enable adhesion development and were then sacrificed. Routine inspection and animal husbandry was performed. This study was performed after approval by "The Israel Board for Animal Experiments" and in compliance with "The Israel Animal Welfare Act".

### TEST PROCEDURE

All tested animals were randomly assigned to respective treatment.

### EXPERIMENTAL DESIGN AND CONDITIONS

Constitution of test groups, number of animals, test material, volume and administration route are shown in Table 16. All of the solutions (control v. fresh) were used at room temperature.

Body weight was measured 3 days before the experiment or at the same day, prior to test administration.

**Table 16:**

| **Group no.** | **Rat no.** | **Test article** | **Dose/Volume (ml/kg)** | **Route** |
|---|---|---|---|---|
| **1** | 3 | CNTRL | 1 ml | Applied on cecum and peritoneum |
| **2** | 3 | FRESH | 1 ml | Applied on cecum and peritoneum |

**TEST PROCEDURES**: rats were treated with the test material by dripping or spraying the test article from a high-gauge needle on the surgically induced adhesion site (the abraded cecum).

### Procedure:

15 days following administration of test articles, animals were terminally anesthetized by pentobarbital. The animals' abdomens were opened and the peritoneum and cecum exposed. Adhesions were graded according to a standard grading scale of 0 (no adhesions) to 4 (persistent adhesions that cannot be removed without damaging the underlying organs), according to standard scoring. Images of the exposed peritoneum, of the cecum, and of any adhesion, were taken and recorded. Samples were collected.

### RESULTS AND DATA

No adverse effects were reported during this study. All animals in both control and test groups gained weight normally. No fever, apathetic behavior, lack of grooming, social isolation behavior, or deaths, were recorded. Table 17 displays the weight gain recorded for all animals in the experimental groups. All animals gained weight normally, and not a single animal lost weight. Average weight gain was 37.5 gr/week (+/- 1.9 gr).

Table 17. Animal body weight is shown in Table 17, where the pre-operative weight was recorded on 21/6/2016, and the post-operative weight was recorded on 28/6/2016 and 6/7/2016. Control group (CNTRL.) receiving saline, treatment (TEST) receiving fresh test material.

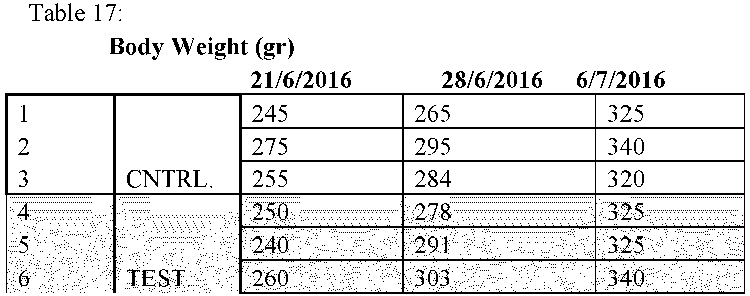

### Results

Table 18 shows the descriptions of all adhesions analyzed in the study.

**Table 18:**

| **Animal No.** | **Group** | **Adhesion length (mm)** | **Location of Adhesion** |
|---|---|---|---|
| 1 | Control | 5 | Fat of bladder to incisional wound |
| 1 | Control | 3 | Omentum to abraded cecum |
| 2 | Control | 3 | Fat of bladder to cecum |
| 3 | Control | 2 | Fat of bladder to incisional wound |
| 4 | Test | 4 | Omentum to incisional wound |
| 5 | Test | NO ADHESIONS | N/A |
| 6 | Test | NO ADHESIONS | N/A |
| *** All adhesions were <1mm thin grade 1 "stripes"** | | | |

Table 19 shows a summary of experimental results.

**Table 19:**

| | **Total No. of adhesions** | **% animals with adhesions** |
|---|---|---|
| Control | 4 | 100% |
| Test | 1 | 33% |

Experimental results are displayed in tables 18 and 19. The cecal abrasion without peritoneal sidewall defect model produced adhesions in all control animals (including 2 adhesions in animal number 1; see, Table 18). These were, however, mild grade 1 adhesions which were all filmy and thin. Area was not possible to calculate as these adhesions presented as thin "strips" of tissue rather than more significant adhesions that can dominate a region overlaying internal organs and/or the peritoneum. Still, the effect of the test material was clearly seen as only one adhesion was detected, in only one of three animals treated with the test article (Table 19).

### CONCLUSIONS

In summary, the composition produced by the kit of the present invention was able to reduce and almost entirely prevent mild adhesions generated by a mild stimulation protocol.

The current test model employed here simulates the many open surgical procedures in which some damage to internal organs may occur, but no intentional damage is done to the peritoneum. These procedures generate clinically meaningful surgical adhesions. Over time, these adhesions can develop, leading to significant side effects. Indeed, all three control animals showed an adhesion (one animal displaying two adhesions). Against this background, administering the test material produced by the kit provided two animals without any adhesions, and only one animal with a single adhesion. Thus, the method using the subject kit was effective in minor as well as major surgical procedures, and has tested well in different severity models. These results are consistent with the results of the previous experiments.

Table 20 shows the biochemical activity of the different factors for test material used in this experiment.

**Table 20:**

| Factor: | Factor II | Factor V | Factor VII | Factor X | Fibrinogen |
|---|---|---|---|---|---|
| Levels relative to input plasma | 506% | 52% | 391% | 454% | 13% |

Absolute concentrations were approximately 4-5-fold for all enzymatic factors (II, VII, X) and about 50% for Factor V. Fibrinogen levels were approximately 13% of the input plasma levels in the concentrated material.

### EXAMPLE 5: CLOTTING ASSAY TO DETERMINE CONCENTRATION OF CLOTTING FACTORS

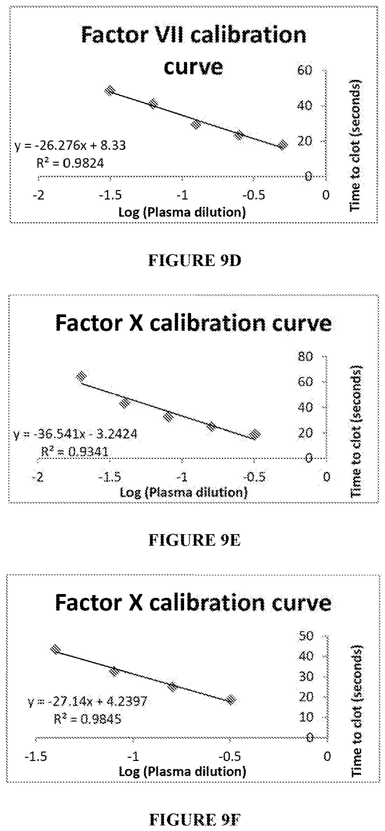

The percent recovery of each clotting factor and associated clotting times were tested, and the following figures are graphs representing the results of these studies: Factor II (Figures 9A and 9B), Factor V (Figure 9C), Factor VII (Figure 9D), Factor X (Figures 9E and 9F), and Fibrinogen (Figures 9G (log curve) and 9H (linear fit)). Once the clotting times were obtained, the amount of each clotting factor was calculated.

### Method:

Human Fresh Frozen Plasma (FFP) was processed using the method presented herein. 150 ml of plasma were concentrated to a volume of 9 mls using an automated FPLC device. This device, while not suitable for use in an OR environment, can nonetheless emulate an automated process. A modified prothrombin time assay using complementation of deficient plasma was used to analyze the levels of the extrinsic system factors (II, V, VII, and X). A Clauss assay was used to analyze the levels of Fibrinogen.

The following parameters were used to obtain eluate (including, e.g., factor II, factor V, factor VII, factor X, or any combination thereof) from the subject kit: loading the AEX column at a rate of 3mL/min, washing the column with a low salt solution (75mM NaCl) at a rate of 5mL/min, eluting at a rate of 1mL/min using a high salt solution (750mM).

The recovered concentrations of clotting factors are as follows:

**Table 21:**

| **Recoveries (average)** | | | **In Diluted Extract** | |
|---|---|---|---|---|
| | **Min** | **Max** | **Amount (Fold over input plasma)** | |
| **FII** | 84% | 101% | 5.6 | 6.7 |
| **FV** | 15% | 28% | 1.0 | 1.9 |
| **FVII** | 100% | | 6.7 | |
| **FX** | 93% | 100% | 6.2 | 6.7 |
| **Fibrinogen** | 8% | 12% | 0.5 | 0.8 |

The results of the clotting times for the standard curve obtained from the input plasma are shown in Table 22:

**Table 22:**

| **Factor II** | | |
|---|---|---|
| **Dilution** | **Log** | **Clotting Time (seconds)** |
| 0.32 | -0.494850022 | 18.1 |
| 0.16 | -0.795880017 | 21.1 |
| 0.08 | -1.096910013 | 28.3 |
| 0.04 | -1.397940009 | 39.5 |
| 0.02 | -1.968970004 | 46.8 |
| 0.01 | -2 | 59.8 |

Tables 23 and 24 show the results of clotting time using various dilutions of recovered factor II when calculated against 5-point and 6-point standard curves generated on the basis of the clotting times for input plasma (see table 22). The wash solution exhibited the longest clotting time as shown in Tables 23 and 24, as the wash solution did not include measurable levels of factor II. However, the dilutions of the eluted factor II exhibited clotting times between 15.6 and 34 seconds, depending on the dilution factor.

**Table 23:**

| | **Dilution** | **Time (seconds)** | **In undiluted extract** | **% Factor II Recovered** |
|---|---|---|---|---|
| **Wash solution** | 1:10 | 1000 | 0% | 0% |
| **Elution** | 1:40 | 15.6 | 1160% | 70% |
| **Elution** | 1:80 | 19.7 | 1660% | 100% |
| **Elution** | 1:160 | 26.8 | 1858% | 111% |
| **Elution** | 1:320 | 34 | 2063% | 124% |
| **Average** | | | | 101% |

**Table 24:**

| | **Dilution** | **Time (seconds)** | **In undiluted extract** | **% Factor II Recovered** |
|---|---|---|---|---|
| **Wash solution** | 1:10 | 1000 | 0% | 0% |
| **Elution** | 1:40 | 15.6 | 875% | 52% |
| **Elution** | 1:80 | 19.7 | 1300% | 78% |
| **Elution** | 1:160 | 26.8 | 1557% | 93% |
| **Elution** | 1:320 | 34 | 1850% | 111% |
| **Average** | | | | 84% |

Prothrombin (FII) levels in a therapeutic extract generated from the concentrated material would therefore (based on an estimate, e.g., table 23 or 24) be between 5.6 and 6.7-fold the concentration in input plasma. This is based on the requirement to reduce the NaCl in the elution solution (750mM) to a concentration approximating twice that of physiological saline (300mM - a 2.5-fold dilution).

Figures 9A and 9B further illustrate the results shown in Tables 23 and 24.

Table 25 shows the standard curve for factor V activity based on the PT complementation assay with Factor V deficient plasma, generated from input plasma. Figure 9C shows the graphical results of Table 25. Dilutions ranging from 0.5 to 0.015625 of the eluateinput plasma displayed clotting times of between 17.4 to 52.7 seconds.

**Table 25:**

| **Factor V** | | |
|---|---|---|
| **Dilution** | **Log** | **Clotting Time (seconds)** |
| 0.5 | -0.301029996 | 17.4 |
| 0.25 | -0.602059991 | 22.1 |
| 0.125 | -0.903089987 | 27.2 |
| 0.0625 | -1.204119983 | 33.4 |
| 0.03125 | -1.505149978 | 45.7 |
| 0.015625 | -1.806179974 | 52.7 |

Table 26 shows the results of clotting time using various dilutions of eluate in the same complementation assay for factor V and the % recovery of factor V from the kit disclosed herein.

**Table 26:**

| | **Dilution** | **Time (seconds)** | **In undiluted extract** | **% Factor V Recovered** |
|---|---|---|---|---|
| **Wash solution** | 1:10 | 52.6 | 14% | 1% |
| **Elution** | 1:10 | 18.7 | 418% | 25% |
| **Elution** | 1:20 | 23.1 | 470% | 28% |
| **Elution** | 1:40 | 29.5 | 408% | 24% |
| **Elution** | 1:80 | 38.8 | 242% | 15% |
| **Average** | | | 432% | 26% |

Table 27 shows the standard curve generated from clotting times using various dilutions of input plasma in a factor VII complementation assay. Figure 9D further shows these results in graphical form (not including dilution 0.015625). Dilutions ranging from 0.5 to 0.015625 of the eluateinput plasma yielded clotting times of between 17.8 and 48.5 seconds.

**Table 27:**

| **Factor VII** | | |
|---|---|---|
| **Dilution** | **Log** | **Clotting Time (seconds)** |
| 0.5 | -0.301029996 | 17.8 |
| 0.25 | -0.602059991 | 23.4 |
| 0.125 | -0.903089987 | 29.5 |
| 0.0625 | -1.204119983 | 41.1 |
| 0.03125 | -1.505149978 | 48.5 |
| 0.015625 | -1.806179974 | 65.2 |

Table 28 shows the results of clotting times in this assay using various dilutions of the elution.

**Table 28:**

| | **Dilution** | **Time (seconds)** | **In undiluted extract** | **% Factor VII Recovered** |
|---|---|---|---|---|
| **Wash solution** | 1:10 | 64.7 | 7% | 1% |
| **Elution** | 1:40 | 20.4 | 1389% | 83% |
| **Elution** | 1:80 | 21.1 | 2613% | 157% |
| **Elution** | 1:160 | 33.7 | 1732% | 104% |
| **Elution** | 1:320 | 43.8 | 1430% | 86% |
| **Average** | | | **1791%** | **107%** |

Table 29 shows the results of the standard curve generated from input plasma using the complementation assay for factor X activity. Figure 9E shows the graph of the results shown in Table 28. Specifically, dilutions ranging from 0.32 to 0.02 yielded clotting times of between 18.7 to 64.5. Figure 9F shows the same results, without the 0.02 dilution point.

**Table 29:**

| **Factor X** | | |
|---|---|---|
| **Dilution** | **Log** | **Clotting Time (seconds)** |
| 0.32 | -0.494850022 | 18.7 |
| 0.16 | -0.795880017 | 25 |
| 0.08 | -1.096910013 | 32.6 |
| 0.04 | -1.397940009 | 43.4 |
| 0.02 | -1.698970004 | 64.5 |

Tables 30 and 31 show the extrapolated activity in the extract for Factor X based on either a 5-point or 4-point standard curve, respectively.

**Table 30:**

| | **Dilution** | **Time (seconds)** | **In undiluted extract** | **% Factor X Recovered** |
|---|---|---|---|---|
| **Wash solution** | 1:10 | 1000 | 0% | 0% |
| **Elution** | 1:40 | 16.3 | 1167% | 70% |
| **Elution** | 1:80 | 22.6 | 1570% | 94% |
| **Elution** | 1:160 | 30.4 | 1921% | 115% |
| **Elution** | 1:320 | 39.6 | 2151% | 129% |
| **Average** | | | **1702%** | **102%** |

**Table 31:**

| | **Dilution** | **Time (seconds)** | **In undiluted extract** | **% Factor X Recovered** |
|---|---|---|---|---|
| **Wash solution** | 1:10 | 1000 | 0% | 0% |
| **Elution** | 1:40 | 16.3 | 1256% | 75% |
| **Elution** | 1:80 | 22.6 | 1554% | 93% |
| **Elution** | 1:160 | 30.4 | 1714% | 103% |
| **Elution** | 1:320 | 39.6 | 1700% | 102% |
| **Average** | | | **1556%** | **93%** |

Table 32 shows the results of clotting time in a Clauss assay in a standard curve using various dilutions of fibrinogen. Figure 9F shows the graph derived from the information in Table 32. Specifically, clotting times between 8.2 and 11 seconds are in connection with dilutions of input plasma ranging from 0.1 to 0.2 eluate.

**Table 32:**

| **Fibrinogen** | | |
|---|---|---|
| **Dilution** | **Log** | **Clotting Time (seconds)** |
| 0.2 | -0.698970004 | 8.2 |
| 0.175 | -0.756961951 | 8.5 |
| 0.15 | -0.823908741 | 9.1 |
| 0.125 | -0.903089987 | 10.6 |
| 0.1 | -1 | 11 |

Table 33 shows the calculated amounts of Fibrinogen in the eluate both in the concentrated 9 mls generated, and in the material diluted to 300mM NaCl concentration (as was discussed for Prothrombin [FII] in this example).

**Table 33:**

| | **Dilution** | **Time (seconds)** | **Extrapolated in original material** | **Recovered** | **Fibrinogen in 2.5 × diluted material** |
|---|---|---|---|---|---|
| **Elution** | 0.1 | 10.1 | 127% | 8% | 51% |
| **Elution** | 0.05 | 16.5 | 204% | 12% | 82% |
| **Elution** | 0.025 | 27.8 | 131% | 8% | 52% |
| **Wash** | 1 | 8.3 | 23% | 2% | |

Conclusions: High recoveries of the extrinsic system coagulation factors can be obtained using the method described herein. These can be originally obtained at very concentrated levels, and diluted to a working concentration as desired that is also reduced in NaCl.

### EXAMPLE 6: MANIFOLD AND KIT

### General description of the kit

The subject device and associated kit are designed to process approximately 150 ml of plasma for infusion by passing it through a disposable pre-gamma irradiated sterile kit. The function of the kit is to extract from this plasma a concentrate of proteins that is to be used in surgery for the prevention of surgical adhesion formation.

The extract is generated by performing an *in situ* bioprocess. The plasma is first passed through an anion exchange (AEX) 5 ml mini-column which binds these proteins selectively out of the mixture of the proteins found in the plasma. Next, the AEX mini-column is washed with approximately 15 ml of wash buffer to purge the plasma trapped within the dead volume of the column and also remove proteins bound less selectively to the AEX matrix within the column. The liquids of these first two steps are collected in a syringe or other vehicle for subsequent disposal as biological waste. In the next step, approximately 10 ml of elution buffer, a buffer containing a high concentration of chloride anions, is passed through the column. The chloride ions serve to detach the proteins via competition for binding sites, so that the proteins are carried with the elution solution out of the column, and into an application syringe, through the action of a valve positioned at the exit from the column. The application syringe contains a volume of dilution buffer (approximately 15 ml) to reduce the concentration of the salt ions. Lastly, an applicator (e.g., a spray head or other device) is then fastened on the application syringe to yield the final product of the kit: an application syringe filled with approximately 25 ml of final active extract to be sprayed into the abdominal cavity of a surgical patient before closing and/or during surgery.

The kit comprises: (i) syringes containing wash and elution solutions/buffers, (ii) sterile syringes for accepting plasma, biological waste products, and final product, (iii) a miniature anion exchange (AEX) column, e.g., but not limited to, the GE HiTrap Q FF 5 ml minicolumn, (iv) valves to control the direction of flow of the different liquids, and (v) separately packaged spray-head or other solution to be attached to the syringe containing the final product for efficient dispersal of the extract.

The AEX column will be prepared by pre-washing and equilibrating the column, washing with 2-5 CVs (column volumes ~ 5 ml) of washing solution, washing with 2-5 CVs of elution solution, and 2-5 CVs of washing solution.

### Description of the device

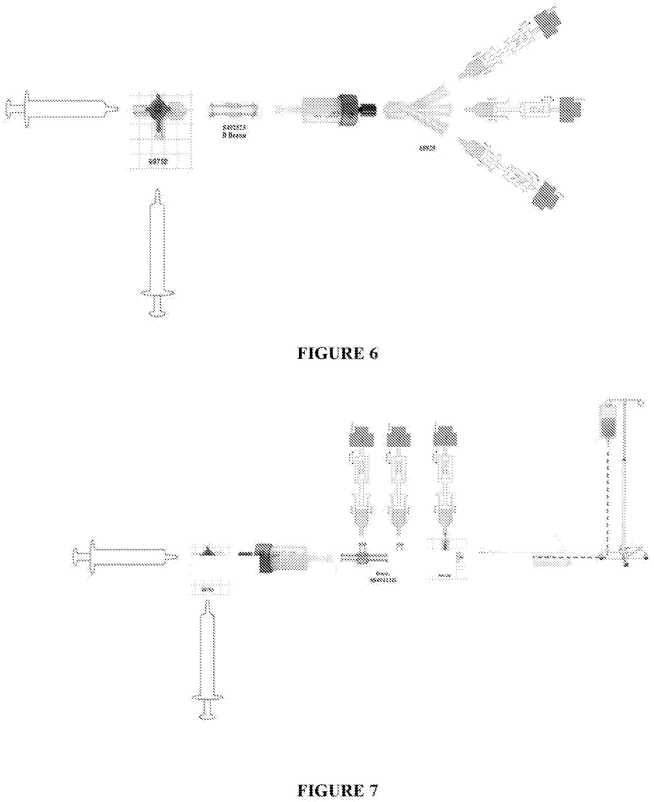

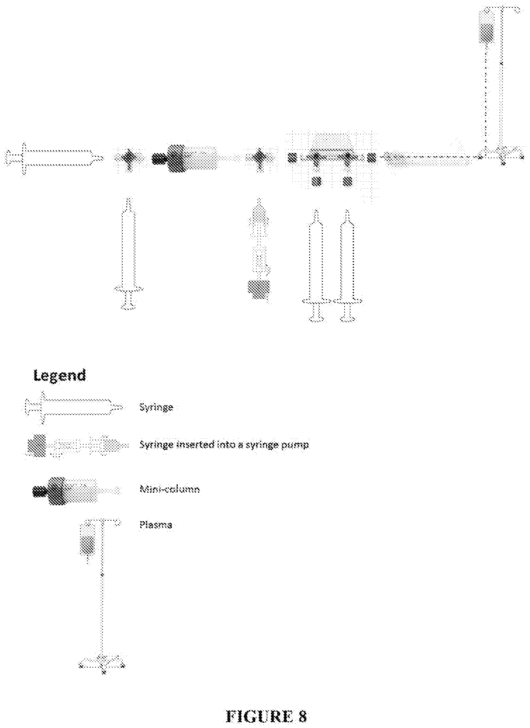

A device, being the operating manifold for the combination of syringes, buffers, and column comprising the assembled kit, can include the following two broad designs: (1) Three syringe pumps for each of the three solutions (1. Plasma, 2. Wash solution and 3. Elution solution) (see designs 1 and 2, Figures 6 and 7) and (2) a syringe pump, a syringe, and valve - liquid can be withdrawn from three separate syringes through a manifold piece, to direct the liquid into the column through a valve (see design 3, Figure 8). Figures 6, 7 and 8 show designs 1, 2 and 3.

Designs 1, 2 and 3 can include a controller unit and any additional required hardware allowing the orderly activation of the syringe pump(s), automatically setting the position of the valves in the system, to ensure that the different solutions are pumped at the required rates, in the pre-established routes, through the disposable kit.

Designs 1, 2 and 3 can include plastic (or any other material) casings and/or fittings and/or stands to allow for insertion of the disposable sterile kit by the user.

Designs 1, 2 and 3 can further include a user interface which enables the user to move between the following modes:
a. Preparation - easy insertion of the kit into the manifold, including the plasma or plasma-containing syringe. This could be achieved, for example, by the device withdrawing and opening the syringe pumps.
b. Operation - the automatic driving of the syringe pumps and valves so as to obtain a pre-determined sequence of events of flowing of fixed volumes of solutions through the mini-column and into the appropriate container (e.g. waste syringe, application syringe) to deliver the protein extract into the application syringe.
c. Emergency stop - This button stops the operation of the system, can disconnect the syringe pumps from the syringes (typically by raising a locking bar holding the syringe in place), and retracts the syringe pumps completely without pushing or withdrawing solutions. This button enables the operator to remove the disposable kit safely and perform any required cleaning in case, as a non-limiting example, of a leak.

The user interface can give such information as time left to finish of process and the current state of the operation (e.g., but not limited to, plasma loading, wash, elution...). Advanced fail-safes such as liquid spill sensors (e.g. by shorting a circuit if liquid has accumulated under valves and connectors), or pressure sensors to allow pressure-regulated flow can be optionally added.

### Kit (design 1, Figure 6)

The kit can include a connector piece that will join the output of the syringes from the syringe pumps into the minicolumn. In this design, (1) a syringe is used to obtain plasma from a plasma bag and is then attached to the connector, or design (2) (Figure 7) a connector piece as described but containing one valve attached at the point of entry of the plasma syringe into the connector, onto which a needle is attached (with plastic cover, as it is typically sold). This needle is then used to impale a plasma bag and open a liquid channel into the connector piece and the rest of the kit (design 2). In design 2, therefore, the plasma becomes continuous with the kit. The syringe pump holding the plasma syringe will therefore withdraw the syringe in order to fill it with plasma from the bag, and the valve is utilized to direct this flow. Once 150 ml have been withdrawn from the bag, the valve will turn to a position that will place the plasma syringe in direct liquid communication with the connector and the other syringes. Figure 7 shows Design 2.

### Kit (design 3)

Design 3 is shown in Figure 8. The different syringes may be connected via a manifold which contains at least: (i) one port for the switch valve into which the syringe pump and working syringe are connected, (ii) one port for the wash buffer syringe, (iii) the port of the elution buffer syringe, and (iv) the port for the plasma containing syringe or needle for impaling a plasma bag (as described for designs 1 (Figure 6) and 2 (Figure 7)). The legend describing Figures 6-8 is shown in Figure 8.

For design 3 (Figure 8), a 3-way valve can join this manifold to the mini-column, and the working syringe that is placed within the syringe pumps. Thus, setting the valve to open a liquid communication channel between the syringe pump and the manifold will enable the syringe pump to withdraw the working syringe thus filling it with the solution selected by the valve position settings on the manifold. Setting the 3-way valve to close this channel and instead open the channel between the working syringe in the syringe pump and the minicolumn will allow the syringe pump to then push this solution into the mini-column. Design 3 further includes the mini-column, as well as a 3- or 4-way valve connected to the exit of the mini-column with at least one port connected to the column exit, one port directed towards a syringe or other container for disposal of plasma and wash solutions containing biological waste, and one port directed towards the application syringe. Optionally, Design 3 can include one port directed towards a syringe or other container for disposal of wash solutions without biologicals (requires a 4-way valve). Lastly, Design 3 can include a spray-head attached to the application syringe after the extract has been generated.

A plasma syringe either separately packaged (e.g. Design 1), or pre-attached (e.g. Design 2). Design 3 may also utilize both options. A wash solution syringe containing sufficient amounts of wash solution (approximately 50 ml), pre-attached to the connector piece, valve or manifold, is included in Designs 1, 2 and 3. Designs 1 and 3 further include an elution solution syringe containing sufficient amounts of elution solution (approximately 50 ml), which is pre-attached to the connector. Designs 1, 2 and 3 include a syringe or other container for wash solutions, pre-attached to the exit valve from the mini-column. Lastly, both Designs 1, 2 and 3 include a syringe or other container for biological waste (plasma and plasma-wash) pre-attached to the exit valve from the mini-column, and an application syringe containing a volume of approximately 15 ml of dilution buffer, with approximately 2-5 ml volume of air (for mixing), pre-attached to the exit valve from the mini-column. Figure 5 shows sample design of the disposable kit.

### Example: Anion Exchange Column Testing

### Testing the capability of a 5 ml HiTrap Q FF column (GE) to generate working ESC.

HiTrap Q FF columns were irradiated at between 50-80 kGrey from a Cobalt source (minimal-maximal actual radiation values guaranteed by the service provider, Sorvan, Israel). Following this irradiation, one column was connected to an FPLC machine (AKTA Start, GE) and was automatically loaded with the following solutions:
1. 3 CVs (15 ml) of wash/equilibration solution [10mM Arginine, 10mM NaCitrate, 50mM NaCl, pH 7.4].
2. 4 CVs (20 ml) of priming/elution solution [10mM Arginine, 10mM NaCitrate, 750mM NaCl, pH 7.4].
3. 3 CVs (15 ml) of wash/equilibration solution [10mM Arginine, 10mM NaCitrate, 50mM NaCl, pH 7.4].
4. The column was then loaded with 150 ml of pool plasma.
5. A wash step ensued, using the wash/equilibration solution (3CVs; 15ml).
6. An elution step was then carried out, using the priming/elution solution (1.8 CVs, 8 ml).

Following the procedure, the elution fraction was tested for activity of the different factors. The following levels were recorded in Table 34:

**Table 34:**

| **Factor** | **% Factor Compared to Input Plasma** |
|---|---|
| FII | 1065% |
| FV | 47% |
| FVII | 1387% |
| FX | 1548% |
| Fibrinogen (FI) | 25% |

### Testing the effect of gamma irradiation on HiTrap Q FF 5 ml column pressure tolerance.

HiTrap Q FF columns were irradiated at between 50-80 kGrey from a Cobalt source (minimal-maximal actual radiation values guaranteed by the service provider, Sorvan, Israel). Following this irradiation, a column was connected to an apparatus designed to maintain pressure within the column for a prolonged duration. The pressure was set to approximately 2 bars, which is 10-fold higher than the max pressure achieved during an ESC production protocol (see above).

The pressure was held for approximately 10-15', during which time there was no pressure reduction indicating no leaks, and no visible leaks of fluid were seen neither. Typically, a dosage used for sterilization is 25 kgrey and does not exceed 40 kgrey.

The testing setup included a syringe pump driving a 10 cc syringe filled with water and connected to a column. On the other side the column was connected to an angio machine with an integral pressure gauge. The syringe pump drove the barrel of the syringe until the resistance gave a pressure of approximately 2 bars as recorded on the pressure gauge in the angio machine. The syringe pump was stopped and the pressure was held within the closed system.

### Example 7: Testing Factor V (Proaccelerin) Recovery Using an Automated Protocol

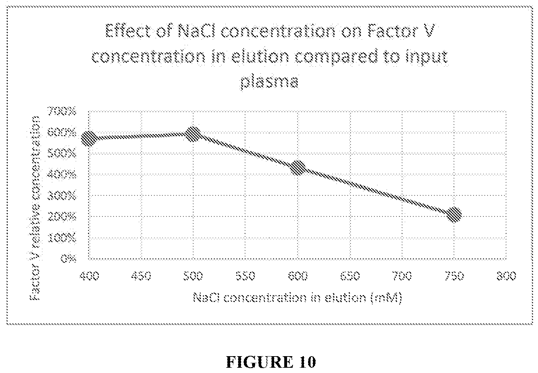

An FPLC was used to perform an automated protocol. Briefly, 150ml of fresh frozen plasma (FFP) were loaded onto a 5 ml HiTrap Q Fast Flow column (GE). A 10 ml elution volume of a citrate buffer (pH 7.4) supplemented with 500, 600, and 750mM NaCl was used to recover the ESC factors from the column. A PT complementation assay performed with Factor V deficient plasma was used to measure Factor V activity levels in the extract against a standard curve generated from input plasma. Factor V activity levels were measured within two hours of the completion of the extraction procedure. Table 35 and Figure 10 show the results of this experiment.

**Table 35:**

| | Factor V concentration in 10 ml elution (compared to input plasma) | Calculated Factor V recovery |
|---|---|---|
| 400mM NaCl elution strength | 570% | 38% |
| 500mM NaCl elution strength | 592% | 39% |
| 600mM NaCl elution strength | 432% | 26% |
| 750mM NaCl elution strength | 210% | 13% |

### Discussion

The activity of Factor V is reduced as a function of NaCl concentration, above 500mM. This is surprising, as elution of proteins from ion exchangers is typically increased with increased salt concentration. Factor V, however, is apparently salt-labile, and its coagulation promoting activity which was measured in this experiment, is reduced with the higher salt concentration.

This is a highly unanticipated result, as it shows a rapid effect (relative, for example, to the hold times in the production of plasma proteins in industrial processes) of intermediate NaCl concentrations (relative, again, to industrial processes) on Factor V stability in the extract. The specific sensitivity of Factor V to NaCl concentrations is unknown.

The rapid Factor V activity reduction may be critical in typical stabilization methods used in the industry such as lyophilization (freeze drying). Lyophilization is a process that takes hours to days. During lyophilization the liquids in pharmaceutical mixtures, typically water, are evaporated. Until water has been nearly completely removed, however, the salt concentrations gradually increase, which can have a detrimental effect on any Factor V found in such a composition (e.g. a PCC composition).

While a number of embodiments of the present invention have been described, it is understood that these embodiments are illustrative only, and not restrictive, and that many modifications may become apparent to those of ordinary skill in the art.

## Claims

1. A purified composition, comprising:
proaccelerin (factor V), prothrombin (factor II), proconvertin (factor VII), and Stuart-Prower factor (factor X),
wherein the concentration of proaccelerin is from 300% to 2000% relative to the concentration in a normal blood plasma sample, wherein the concentration is determined by extrapolating the activity of proaccelerin using a clotting assay and plotting a standard curve;
wherein the concentration of each one of proconvertin, prothrombin and Stuart-Prower factor is from 500% to 2500% or from 150% to 2000% relative to the concentration in a normal blood plasma sample; and
wherein the composition is a fibrinogen-depleted plasma-derived concentrate.

2. The composition of claim 1, wherein the composition comprises 1 - 5% fibrinogen relative to total amount of fibrinogen in the blood plasma.

3. The composition of claims 1 or 2 for use in reducing adhesion severity by topical application at a surgical site in a subject undergoing surgery, wherein the composition is an aqueous composition.

## Patentansprüche

1. Gereinigte Zusammensetzung, aufweisend:
Proaccelerin (Faktor V), Prothrombin (Faktor II), Proconvertin (Faktor VII) und Stuart-Prower-Faktor (Faktor X),
wobei die Konzentration von Proaccelerin von 300% bis 2000% relativ zur Konzentration in einer normalen Blutplasmaprobe beträgt, wobei die Konzentration durch Extrapolation der Aktivität von Proaccelerin unter Verwendung eines Gerinnungsassays und Erstellung einer Standardkurve bestimmt wird;
wobei die Konzentration jedes von Proconvertin, Prothrombin und Stuart-Prower-Faktor von 500% bis 2500%, oder von 150% bis 2000%, relativ zur Konzentration in einer normalen Blutplasmaprobe beträgt; und
wobei die Zusammensetzung ein Fibrinogen-depletiertes, aus Plasma gewonnenes Konzentrat ist.

2. Zusammensetzung nach Anspruch 1, wobei die Zusammensetzung 1-5% Fibrinogen relativ zur Gesamtmenge an Fibrinogen im Blutplasma aufweist.

3. Zusammensetzung nach Anspruch 1 oder 2 zur Verwendung in der Reduktion der Adhäsions-Schwere durch topische Anwendung an einer chirurgischen Stelle bei einem Subjekt, der sich einer Operation unterzieht, wobei die Zusammensetzung eine wässrige Zusammensetzung ist.

## Revendications

1. Une composition purifiée, comprenant :
de la proaccélérine (facteur V), de la prothrombine (facteur II), de la proconvertine (facteur VII) et le facteur Stuart-Prower (facteur X),
la concentration en proaccélérine étant de 300 % à 2000 % par rapport à la concentration dans un échantillon de plasma sanguin normal, la concentration étant déterminée en extrapolant l'activité de la proaccélérine en utilisant un test de coagulation et en traçant une courbe standard ;
la concentration de chacun parmi la proconvertine, la prothrombine et le facteur Stuart-Prower allant de 500 % à 2500 % ou de 150 % à 2000 % par rapport à la concentration dans un échantillon de plasma sanguin normal ; et
la composition étant un concentré dérivé du plasma appauvri en fibrinogène.

2. La composition selon la revendication 1, la composition comprenant de 1 à 5 % de fibrinogène par rapport à la quantité totale de fibrinogène dans le plasma sanguin.

3. La composition selon les revendications 1 ou 2 pour une utilisation dans la réduction de la sévérité de l'adhérence par application topique sur un site chirurgical chez un sujet subissant une intervention chirurgicale, la composition étant une composition aqueuse.
